(19) Europäisches Patentamt
European Patent Office
Office européen des brevets

(11) EP 0 597 300 B1

(12) **EUROPÄISCHE PATENTSCHRIFT**

(45) Veröffentlichungstag und Bekanntmachung des Hinweises auf die Patenterteilung:
**11.09.1996 Patentblatt 1996/37**

(51) Int. Cl.$^6$: **B01D 71/06**, B01D 67/00, B01D 61/00, B01D 53/22

(21) Anmeldenummer: **93117131.8**

(22) Anmeldetag: **22.10.1993**

(54) **Stoffspezifische Trägersubstanzen enthaltende Polymermembran, Verfahren zu deren Herstellung und deren Verwendung zur Trennung von Stoffgemischen**

Polymer membrane containing material specific carriers, process for its production and its utilisation for separating substance compositions

Membrane de polymères contenant des supports spécifiques à la matière, procédé de sa production et son utilisation pour la séparation de compositions de substances

(84) Benannte Vertragsstaaten:
**DE FR GB NL**

(30) Priorität: **11.11.1992 DE 4238097**

(43) Veröffentlichungstag der Anmeldung:
**18.05.1994 Patentblatt 1994/20**

(73) Patentinhaber: **FRAUNHOFER-GESELLSCHAFT ZUR FÖRDERUNG DER ANGEWANDTEN FORSCHUNG E.V.
80636 München (DE)**

(72) Erfinder:
- **Bauer, Bernd
D-70569 Stuttgart (DE)**
- **Strathmann, Heiner, Prof. Dr.
D-72076 Tübingen (DE)**
- **Schulenberg-Schell, Helmut, Dr.
D-71263 Weil der Stadt (DE)**

(56) Entgegenhaltungen:
**EP-A- 0 098 731**
**EP-A- 0 154 247**
**DE-A- 3 112 937**
**DE-A- 3 740 871**
**US-A- 3 625 734**

EP 0 597 300 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Rank Xerox (UK) Business Services
2.13.4/3.4

## Beschreibung

Die Erfindung betrifft stoffspezifische Trägersubstanzen enthaltende Polymermembranen (Carrier-Membranen), ein Verfahren zu deren Herstellung und deren Verwendung zur Trennung von molekularen Stoffgemischen.

Das stoffspezifische Trennen von molekularen Gas- oder Flüssigkeitsgemischen ist ein zentrales Problem in allen Bereichen der technischen Chemie, der Lebensmittelindustrie, der Pharmazie sowie der Rohstoffgewinnung. Innerhalb der verschiedenen Prozesse, die heute für die Stofftrennung zur Verfügung stehen, spielen Membrantrennverfahren eine ganz zentrale Rolle. Um mit ihnen Produktionsprozesse wirtschaftlich zu gestalten, ist es erforderlich, daß die eingesetzten Membranen einen möglichst hohen flächenspezifischen Durchsatz und eine möglichst hohe Selektivität aufweisen. Diese Anforderungen sind insbesonders dann schwierig zu erfüllen, wenn Stoffe getrennt werden sollen, die sich nur wenig in ihren physikalischen und chemischen Eigenschaften unterscheiden, wie dies z.B. bei Stickstoff und Sauerstoff oder bei der homologen Reihe von n-Alkoholen der Fall ist.

Die meisten heute technisch genutzten Membranen bestehen aus Polymeren und besitzen eine symmetrische oder asymmetrische, mehr oder weniger poröse Struktur. Die stofftrennenden Eigenschaften dieser Membranen werden entweder durch die Größe der Poren oder durch die Löslichkeit und Diffusivität der verschiedenen Komponenten in der Matrix des Membranpolymers bestimmt. Da die geometrischen Abmessungen verschiedener Stoffe, deren Löslichkeit und auch deren Diffusionskoeffizient in einer Polymermatrix oft nicht sehr unterschiedlich sind, ist in vielen Fällen eine Trennung mit herkömmlichen Polymermembranen schwierig und wirtschaftlich häufig uninteressant. Bei Flüssigmembranen wird anstatt eines Feststoffes eine Flüssigkeit als Membran verwendet, da hier die Diffusionskoeffizienten (D) um Größenordnungen höher sind als in Polymeren (Feststoffen). Damit wird eine Erhöhung des transmembranen Flusses und somit der Permeabilität festgestellt.

| | | |
|---|---|---|
| Zum Vergleich: | - Polymer | : D = $10^{-11}$ bis $10^{-14}$ $m^2/s$ |
| | - Flüssigkeit | : D = $10^{-9}$ bis $10^{-10}$ $m^2/s$ |

Jedoch wird die Diffusion z.B. sowohl für Sauerstoff als auch für Stickstoff in gleichem Maße erhöht, da die beiden Gase praktisch den selben Durchmesser besitzen. Weiterhin sagt die Stokes-Einstein-Gleichung voraus, daß beide Gase ungefähr den gleichen Diffusionskoeffizienten in allen Medien besitzen, was auch weitgehend beobachtet wurde. Deshalb kann eine Trennung dieser beiden Gase nur auf deren unterschiedliche Löslichkeiten im Polymer zurückgeführt werden. Zur Trennung dieser beiden Gase müssen die Flüssigkeiten also entweder unterschiedliche Löslichkeiten für diese aufweisen, oder ihr werden stoffspezifische Trägersubstanzen, sog. Carrier-Substanzen, zugesetzt, welche befähigt sind, eine der Komponenten, z.B. Sauerstoff, spezifisch binden.

Eine wesentlich bessere Trennung von chemisch und physikalisch sehr ähnlichen Stoffen läßt sich also mit Hilfe stoffspezifischer Trägersubstanzen erreichen, wie sie z.B. in Flüssigmembranen enthalten sind. Bei den Flüssigmembranen unterscheidet man zwischen dem Emulsionstyp und den Matrix gestützten Flüssigmembranen. Emulsionsmembranen bestehen z.B. aus zwei durch Öl-Phasen getrennte Wasserphasen, wobei sich hier die Probleme von Extraktionstechniken, d.h. schlechte Phasentrennung und geringe Stabilität der Phasen, zeigen. Bei den gestützten Flüssigmembranen, welche z.B. für die Gastrennung verwendet werden, wird eine Flüssigkeit aufgrund von Kapillarkräften z.B. in der Porenstruktur einer Polymermatrix fixiert. Solche Flüssigmembranen werden üblicherweise dadurch hergestellt, daß man einen Träger, z.B. eine poröse Folie oder eine Platte mit dem Flüssigmembranmaterial tränkt. Dabei übt die poröse Struktur des Trägers keine trennende Funktion aus, sondern hat nur die Aufgabe, die in ihren Poren eingeschlossene Flüssigkeit mechanisch zu stabilisieren. Die eigentliche Stofftrennung wird von den selektiven Trägersubstanzen in der Flüssigkeit übernommen.

Als poröse Träger eignen sich z.B. Ultrafiltrationsmembranen, die durch ihre geringen Porendurchmesser hohe Kapillarkräfte besitzen und die somit in der Lage sind, die Flüssigkeit auch bei höheren Drücken in der Membran zu halten. Die Flüssigkeit muß dabei bestimmte Voraussetzungen erfüllen. Diese sind:

- geringer Dampfdruck, damit sie sich während des Betriebes nicht verflüchtigt;
- günstige Selektivität bezüglich einer Komponente;
- Benetzbarkeit der Poren des Trägers und chemische Verträglichkeit für den Träger;
- niedrige Viskosität, da die Diffusionskoeffizienten von Gasen umgekehrt proportional zur Viskosität sind.

Der Stofftransport in Flüssigmembranen erfolgt derart, daß die Trägersubstanz in der Membran zirkuliert und an der dem Rohgemisch zugewandten Seite der Membran, der Feed-Seite, einen bestimmten Stoff, z.B. Sauerstoff, selektiv und reversibel aufnimmt, per Diffusion in der Flüssigkeit durch die Membran transportiert und auf der Permeatseite der Membran wieder abgibt. Der unbeladene Carrier diffundiert dann infolge des Konzentrationsgradienten von der

Permeat- zur Feed-Seite zurück. Die hohe Selektivität von Flüssigmembranen beruht darauf, daß die Trägersubstanz ausschließlich mit der zu transportierenden Komponente koppelt und alle anderen Stoffe des Gemisches nicht in die Flüssigkeit eindringen und somit durch die Membran nicht permeieren können. Im Falle der Stickstoff/Sauerstoff-Trennung ist eine Komponente, z.B. der Stickstoff, nicht befähigt, mit dem Carrier eine Bindung einzugehen.

Flüssigmembranen in Verbindung mit stoffspezifischen Trägersubstanzen zeichnen sich durch sehr hohe Spezifitäten für eine Komponente sowie durch hohe Permeabilitäten aus. Dies gilt besonders für die Trennung von Gasen, wie z.B. Stickstoff und Sauerstoff, einem Stofftrennproblem von ganz besonderer wirtschaftlicher Bedeutung. Durch die Verwendung einer selektiven Trägersubstanz, z.B. eines Kobalt-Salen-Komplexes, in Verbindung mit einer Flüssigmembran lassen sich ganz enorme Leistungssteigerungen in der Permeabilität und Selektivität von Polymeren erzielen. Flüssigmembranen mit einem Kobalt-Salen-Komplex zeigen eine ca. 10 bis 20 mal höhere Selektivität und besitzen eine ca. 10 mal höhere Permeabilität als herkömmliche Polymermembranen. Außer dem Kobalt-Komplex sind in der Literatur noch eine Reihe anderer Schwermetallkomplexe beschrieben, die über ausgezeichnete selektive Stofftransporteigenschaften verfügen, und zwar nicht nur für Gasgemische, sondern auch z.B. für bestimmte Isomerengemische oder für Alkan-Alken-Gemische. Damit bietet die Flüssigmembrantechnik in Verbindung mit selektiven Trägersubstanzen eine ganz erhebliche technische und wirtschaftliche Verbesserung bei der Nutzung von Membranen zur Trennung von molekularen Gemischen.

Flüssigmembranen sind z.B. aus den folgenden Druckschriften bekannt: EP 0098731 B1, EP 0176986 A2, EP 0186182 A2, EP 0213744 A2, US 3,625,734, US 4,705,544, US 4,174,374 und US 4,710,205.

Allerdings besitzen Matrix gestützte Flüssigmembranen zwei ganz gravierende Nachteile, die bis heute einer technischen Nutzung im industriellen Maßstab entgegenstanden. Diese sind die sehr schlechten mechanischen Eigenschaften sowie ihre im Vergleich zu asymmetrischen Polymermembranen große Dicke. Während bei konventionellen Membranen die eigentliche selektive Schicht eine Dicke von oft nur wenigen Nanometern hat, verfügen trägergestützte Flüssigmembranen im allgemeinen über die gleiche Dicke wie die poröse Trägerschicht. Diese ist aber selten dünner als 20 bis 50 Mikrometer. Somit sind Flüssigmembranen im allgemeinen ca. 100 bis 1000 mal dicker als asymmetrische Polymermembranen, womit der Vorteil der höheren Permeabilität von Flüssigmembranen gegenüber Polymermembranen durch deren größere Dicke wieder verloren geht, so daß im allgemeinen mit Flüssigmembranen keine höheren Permeationsstromdichten zu erzielen sind.

Die geringe mechanische Stabilität der Flüssigmembranen wirkt sich in zweifacher Hinsicht negativ aus: einerseits können Flüssigmembranen nur mit sehr geringen hydrostatischen Drücken beaufschlagt werden, da andernfalls die Flüssigkeit mit der selektiven Trägersubstanz aus der porösen Stützstruktur herausgedrückt werden würde. Somit sind hohe hydrostatische Druckdifferenzen, wie sie bei asymmetrischen Porenmembranen üblich sind, bei Flüssigmembranen nicht anwendbar. Ferner weisen die Flüssigkeiten, die zur Aufnahme von selektiven Trägersubstanzen geeignet sind, einen noch erheblichen Dampfdruck oder auch erhebliche Löslichkeit in den Außenphasen auf, so daß die flüssige Phase der Membran nach mehr oder weniger kurzer Zeit aus der porösen Unterstruktur eluiert, und somit die Lebensdauer von Flüssigmembranen begrenzt ist. Versuche, selektive Trägersubstanzen chemisch über Seitenketten an ein Trägerpolymer zu binden, waren bisher nicht erfolgreich, da dadurch der eigentliche Transportmechanismus, nämlich die Diffusion der zu transportierenden Komponente mit dem Träger in der flüssigen Membranphase, empfindlich gestört und damit die Permeabilität häufig verschwindend gering wird.

Aus der EP 0154247 B1 sind Membranen aus Vinylpolymeren bekannt, bei denen Sauerstoff übertragende Gruppen kovalent an die Polymerkette gebunden sind.

Für die Gastrennung, wie z.B. für die Abtrennung von Sauerstoff, werden als stoffspezifische Trägersubstanzen Metall-Chelate, vorwiegend Co-Salen- und Co-Porphin-Verbindungen eingesetzt. Hier ist $Co^{2+}$ als Zentralatom von fünf elektronenspendenden Liganden koordiniert, wobei einen der Liganden eine sog. Axialbase bildet. Der molekulare Sauerstoff wird direkt vom $Co^{2+}$-Atom reversibel gebunden. Die für die Sauerstoff-Abtrennung verwendeten stoffspezifischen Trägersubstanzen haben die folgenden Anforderungen zu erfüllen, um einen trägergebundenen Sauerstoff-Transport zu gewährleisten:

- Chemische Stabilität der Komplexe;
- Reversibilität der Sauerstoff-Aufnahme;
- Hohe Geschwindigkeit der Sauerstoff-Aufnahme und -Abgabe;
- Mobilität der Komplexe im flüssigen und polymeren Teil der Membran.

Diese Anforderungen gelten ganz analog für alle Carrier.

Die chemische Stabilität der Co-Komplexe ist vorallem durch deren Autoxidation limitiert. Autoxidation erfolgt vornehmlich bei steigenden Temperaturen und höherem Wassergehalt der Lösung. Zu einer irreversiblen Bindung des Sauerstoff-Moleküls kann es z.B. dadurch kommen, daß das Co(II) zum Co(III) oxidiert wird, indem der Co-Komplex in die Superoxo-Form ($O_2^-$) übergeht und mit einem zweiten sauerstoffreichen Carrier-Molekül zur Peroxo-Verbindung ($O_2^{2-}$) dimerisiert. Die PeroxoVerbindung ist dann in der Lage, sich irreversibel in die Oxo-Form ($O^{2-}$) umzuwandeln.

Aufgabe der vorliegenden Erfindung ist es deshalb, Polymermembranen für Membrantrennverfahren zur Trennung von molekularen Flüssigkeits- oder Gasgemischen zur Verfügung zu stellen, die zur Erhöhung der Selektivität mit stoffspezifischen Trägersubstanzen ausgestattet sind. Dabei sollen die stoffspezifischen Trägersubstanzen entweder als solche (Fest-Carrier-Membran) oder in gelöster Form (Flüssigmembran) in der Membran enthalten sein. Für den Fall, daß die stoffspezifischen Trägersubstanzen als Lösung in der Membran vorliegen, sollen die erfindungsgemäßen Membranen nicht über die Nachteile von Flüssigmembranen gemäß dem Stand der Technik verfügen, sondern sie sollen vielmehr die Vorteile von Polymermembranen und Flüssigmembranen in sich vereinen. Die erfindungsgemäßen Membranen sollen hohe Selektivitäten mit hohen Permeabilitäten und geringen Membrandicken verbinden, und sie sollen hohe mechanische Stabilitäten aufweisen, insbesondere hinsichtlich des beaufschlagten hydrostatischen Druckes. Ferner sollen die Membranen lange Standzeiten besitzen, insbesondere bezüglich der Stabilität der Flüssigkeiten, d.h. das Verdampfen der Flüssigkeit bzw. deren Lösungsmittels soll stark vermindert sein.

Membranen für die Stickstoff/Sauerstoff-Trennung sollen außerdem stoffspezifische Trägersubstanzen enthalten, die hinsichtlich der Oxidationsbeständigkeit gegenüber Sauerstoff im Vergleich zum Stand der Technik stark verbessert sind.

Das Herstellungsverfahren für die erfindungsgemäßen Membranen soll einfach und universell anwendbar sein, universell sowohl hinsichtlich der verwendbaren stoffspezifischen Trägersubstanzen als auch hinsichtlich deren Aggregatzustandes, d.h. diese sollen sowohl in fester als auch in gelöster Form in der Membran enthalten sein. Aufgabe der Erfindung ist es somit ferner, ein Verfahren zu entwickeln, mit dem sowohl Fest-Carrier-Membranen als auch Flüssigmembranen zur Verfügung gestellt werden können.

Gelöst wird diese Aufgabe durch stoffspezifische Trägersubstanzen (Carrier) enthaltende Polymermembranen (Carrier-Membranen), die durch ein Trocken-Naß-Phaseninversionsverfahren erhältlich sind, indem man aus einer homogenen, flüssigen Polymerlösung, die

- zu 8 bis 35 Gew.-% aus einem organischen Polymer besteht,
- zu 20 bis 60 Gew.-% aus einem leichtflüchtigen Lösungsmittel,
- zu 15 bis 80 Gew.-% aus einem schwerflüchtigen Lösungsmittel und
- zu 1 bis 20 Gew.-% aus einem oder mehreren stoffspezifischen Trägersubstanzen, bzw.
- zu 10 bis 60 Gew.-% aus einer 0.02 bis 0.5 molaren Lösung der stoffspezifischen Trägersubstanzen in einem schwerflüchtigen, mit dem Fällungsmittel für die Phaseninversion nicht mischbaren Lösungsmittel, und gegebenenfalls
- zu 5 bis 15 Gew.-% aus einem Nichtlösungsmittel (Fällmittel),

einen Polymerfilm fertigt, daraus das leichtflüchtige Lösungsmittel entfernt und den resultierenden Polymerfilm mittels eines Fällmittels einer Phaseninversion unterzieht.

Bei der Herstellung von Polymermembranen nach einem Phaseninversionsmechanismus wird eine homogene, flüssige Polymerlösung durch Zugabe eines Fällmittels in ein Zweiphasen-System überführt. Dabei entsteht eine feste, polymerreiche Phase, die das Membrangerüst, die Membranmatrix bildet, und eine flüssige, polymerarme Phase, die die Membranporen formt. Durch die Anwendung des Phaseninversionsprozesses werden aus der Polymerlösung poröse Strukturen erhalten, die in Abhängigkeit von den Fällbedingungen symmetrisch oder asymmetrisch sind, und deren Porengröße von 10 µm bis zu wenigen Nanometern varriierbar ist.

Überraschenderweise wurde festgestellt, daß mit dem erfindungsgemäßen Verfahren Membranen zur Verfügung gestellt werden, die hohe Selektivitäten für eine Komponente mit hohen Permeabilitäten, hohen mechanischen Eigenschaften und langen Standzeiten verbinden.

Durch die Anwendung einer sog. Trocken-Naß-Phaseninversion werden erfindungsgemäße Carrier-Membranen mit integral-asymmterischer Struktur erhalten. Überraschenderweise wurde festgestellt, daß durch die Anwendung des erfindungsgemäßen Verfahrens Membranen entstehen, deren Oberfläche aus einer dichten aber dünnen Polymerschicht besteht, und deren Unterstruktur porös und schaumartig ist.

Die zur Herstellung der erfindungsgemäßen Membranen verwendete Polymerlösung enthält neben einem schwerflüchtigen Lösungsmittel zusätzlich ein leichtflüchtiges Solvens, welches nach der Erzeugung des Polymerfilms durch eine erzwungene Konvektion, z.B. durch Abdampfen, aus dem Polymerfilm entfernt wird. Der verbleibende Polymerfilm stellt dann eine Lösung des Polymers nur mehr in dem schwerflüchtigen Lösungsmittel dar. Durch diese erzwungene Konvektion (Trocken-Phaseninversion) kommt es an der Oberfläche des gefertigten Polymerfilms zur Ausbildung einer homogenen, dichten Phase, d.h. an der Oberfläche des Polymerfilms kommt es zur Bildung einer dünnen aber dichten Oberflächenschicht. Erzeugt wird diese dichte, dünne Oberflächenschicht durch das schnelle Entfernen des leichtflüchtigen Lösungsmittels aus der Polymerlösung bzw. aus dem Polymerfilm, so daß das verbleibende System in eine Mischungslücke tritt. Da es sich hier bei der Trocken-Phaseninversion in Folge der guten Flüchtigkeit des leichtflüchtigen Lösungsmittels um eine schnelle Phaseninversion handelt, verbleibt dem Polymer an der Filmoberfläche wenig Zeit, sich zu größeren Agglomeraten zusammenzulagern und es entsteht eine dicht gepackte Phase. Durch den steilen Gradient im Aktivitätskoeffizient kommt es außerdem zu einer Bewegung des Polymers senkrecht zur Polymerlösung

in Richtung Oberfläche. Daraus resultiert eine Anreicherung des Polymers in den oberen Schichten, welche die Ausbildung der dichten Oberflächenschicht begünstigt.

Bei der anschließenden Naß-Phaseninversion mit einem Fällmittel, z.B. in einem Fällbad, wird eine Fällung in den unteren Schichten des Polymerfilms herbeigeführt. Durch die bereits gebildete dichte Schicht an der Oberfläche wird jedoch die Austauschgeschwindigkeit des schwerflüchtigen Lösungsmittel mit dem Fällmittel herabgesetzt, so daß flachere Konzentrationsprofile resultieren und eine schaumartige Unterstruktur hervorgerufen wird.

Die Austauschgeschwindigkeit von Lösungsmittel und Fällmittel wird zum eigentlichen geschwindigkeitsbestimmenden Schritt für das Eindringen des Fällmittels in den Polymerfilm. Damit wird die Diffusion des Fällmittels in den Polymerfilm erniedrigt, und es herrschen ähnliche Verhältnisse wie bei der Fällung aus der Gasphase. Dies bedeutet, daß die Konzentrationsprofile flacher werden und eine statistische über den Membranquerschnitt verteilte Entmischung stattfindet, die zu einer statistisch verteilten Porenstruktur führt. Dabei sind die Poren unterhalb der dichten Oberflächenschicht in ihrer Größe entweder sehr einheitlich, oder der Porendurchmesser nimmt von der Ober- zur Unterseite hin graduell zu. Welcher von beiden Strukturtypen gebildet wird, hängt davon ab, wie dicht die Haut an der Oberfläche ist, d.h. ob in der Unterstruktur nach Ausbildung der Haut die Konzentrationsprofile des Fällmittels über den gesamten Membranbereich völlig flach sind oder ob ein Gradient vorhanden ist, z.B. durch eine undichte Haut.

Tritt ein Konzentrationsgradient des Fällmittels in der Unterstruktur auf, z.B. durch rasches Eindiffundieren des Fällmittels in das Polymer infolge Undichtigkeiten in der Haut, so werden unterschiedliche Porengrößen in Abhängigkeit des Gradienten gebildet. Durch rasches Eindiffundieren von Fällmittel verbleibt dem Polymer direkt unterhalb der dichten Oberflächenschicht weniger Zeit und das Polymer lagert sich nur zu kleinen Bereichen zusammen und bildet eine feine Porenstruktur aus. In der Unterseite des Films dagegen verbleibt dem Polymer mehr Zeit für die Zusammenlagerung zu größeren Bereichen, da das Fällmittel einen weiteren Weg zurücklegen muß. Durch diese nach unten größer werdende Aufenthaltszeit des Polymers zwischen Fällung und Verfestigung werden Poren mit unterschiedlichen Radien gebildet. Infolge der Anreicherung des Polymers in den oberen Schichten während der Trocken-Phaseninversion wird weiterhin eine Verarmung der polymerreichen Phase in den Unterschichten eingeleitet, welche eine grobporige Struktur verursacht.

Abbildung 1 zeigt den Aufbau der erfindungsgemäßen Carrier-Membranen.

Die erfindungsgemäßen Carrier-Membranen enthalten die stoffspezifischen Trägersubstanzen (Carrier) entweder in Form von Lösungen (Flüssigmembranen) oder in fester Form verteilt in der Polymermatrix (Fest-Carrier-Membranen). Überraschenderweise wurde festgestellt, daß sich im Falle der erfindungsgemäßen Flüssigmembranen die Carrier-Lösung in der grobporigen Unterstruktur der Membran befindet, die Carrier-Lösung also quasi in den Membranporen inkapsuliert ist. Da die Carrier-Lösung in den erfindungsgemäßen Membranen in geschlossenen Poren inkapsuliert ist, bieten die erfindungsgemäßen Flüssigmembranen den großen Vorteil, daß sich das Lösungsmittel der Carrier-Lösung im Gegensatz zu den bisher üblichen Porenmembranen aus den Poren nicht mehr verflüchtigen kann. Lösungsmittelverluste können nur mehr durch Diffusion des Lösungsmittels durch das Polymer auftreten, wobei diese Diffusion selbstverständlich wesentlich langsamer abläuft als das Verdampfen des Lösungsmittels aus den Poren, so daß die Standzeiten der erfindungsgemäßen Flüssigmembranen gegenüber dem Stand der Technik erheblich gesteigert werden konnten.

Ferner wurde überraschenderweise festgestellt, daß sich im Falle von Fest-Carrier-Membranen der Carrier in der dünnen aber dicht gepackten Phase an der Membranoberfläche anreichert. Diese dünne, selektive Oberflächenschicht fungiert als eigentliche Membran und besteht aus einer homogenen Polymerphase, welche als Lösungsmembran wirkt.

Ein weiterer Vorteil der erfindungsgemäßen Membranen besteht darin, daß infolge der dünnen, selektiven Oberflächenschicht hohe transmembrane Flüsse erreicht werden, die nur geringe Drücke als treibende Kraft erforderlich machen, so daß bei Einsatz der erfindungsgemäßen Membranen Membrantrennverfahren kostengünstiger werden.

Ein weiterer Vorteil des erfindungsgemäßen Verfahrens besteht darin, daß damit stabile Membranen mit einer hohen Porosität von > 0.4 gefertigt werden können. Da im Falle der erfindungsgemäßen Flüssigmembranen die Poren mit einer Carrier-Flüssigkeit gefüllt sind, bedeutet dies, daß mit einer hohen Porosität der Membran auch eine hohe Selektivität verbunden ist, denn je größer das Porenvolumen der Membran, desto mehr Carrier-Flüssigkeit ist inkapsuliert.

Die erfindungsgemäßen Carrier-Membranen werden zum stoffspezifischen Trennen von gasförmigen oder flüssigen molekularen Gemischen eingesetzt. Am Beispiel der Stickstoff/Sauerstoff-Trennung wird die Wirkungsweise einer erfindungsgemäßen Flüssigmembran näher erläutert, wobei die stoffspezifischen Trägersubstanzen für den selektiven Sauerstofftransport durch die Membran ausgelegt sind. Damit ein erleichterter Sauerstofftransport in der mikroinkapsulierten Flüssigmembran stattfindet, muß die Membran eine möglichst hohe Porosität (> 0.4) besitzen, damit möglichst viel Carrier-Flüssigkeit in der Membran inkapsuliert ist. Erst ab dieser hohen Porosität ist der Stickstofftransport über das Polymer nach dem Henry-Gesetz soweit zurückgedrängt, daß er nur noch eine untergeordnete Rolle spielt.

Die Verminderung des Stickstofftransportes wird zum einen durch dessen niedere Diffusionsgeschwindigkeit im Polymer und zum anderen durch die hohe Porosität im Polymer verursacht, denn durch die hohe Porosität verlängert sich der Diffusionsweg des Stickstoffes durch die Polymermatrix erheblich, da dieser um die mit Carrier-Flüssigkeit gefüllten Poren diffundiert. Entsprechend dem Umwegfaktor $\tau$ erfährt der Stickstoff eine scheinbar größere Membrandicke und benötigt somit mehr Zeit für den Transport durch die Membran. Dies hat zur Folge, daß durch eine hohe Porosität und einem damit verbundenen höheren Flüssigkeitsanteil in der Membran die Selektivität entscheidend erhöht werden konnte.

Die konkurrierenden Gasströme in einer Flüssigmembran, Diffusion in der Polymermatrix und in der Flüssigkeit, können in Analogie zum Ohm'schen Gesetz und den Kirchhoff'schen Gesetzen anhand eines Widerstandmodells betrachtet werden. Abbildung 2 zeigt das Ersatzschaltbild für die Teilströme durch das Polymer und durch die Flüssigkeit nach dem Widerstandsmodell.

Die Gase Sauerstoff und Stickstoff können nun herkömmlich über die Polymermatrix transportiert werden oder aber der Sauerstoff kann über die Trägerflüssigkeit diffundieren. Dabei stellen die einzelnen Teile der Membran (Trägerflüssigkeit und Polymermatrix) unterschiedliche Widerstände dar, die dem Stofftransport entgegengerichtet sind, wobei der Widerstand der Trägerflüssigkeit natürlich erheblich geringer ist als der der Polymermatrix. Je nach Aufbau der Membran sind die Widerstände parallel oder in Serie geschaltet. Beim Transport über die Trägerflüssigkeit setzt sich der Gesamtwiderstand aus der Trägerflüssigkeit und dem die Poren bildenden Polymer zusammen. Für die Diffusion der Gase durch das Polymer ist nur der Widerstand der Polymermatrix ausschlaggebend.

Die Permeabilität einer Membran gegenüber einer Komponente i im Gasgemisch ist proportional zum Leitwert und zur Dicke z der Membran.

$$P_i = \frac{1}{\Delta z} \cdot \left[\frac{1}{R_T + R_{S_1}} + \frac{1}{R_{S_2}}\right]$$

Die Widerstände für den Trägertransport $R_T$ und für die Diffusion durch das Matrix-Polymer

$$R_{S_1} \text{ und } R_{S_2}$$

sind durch folgende Gleichungen beschrieben:

$$R_T = \frac{r_T}{\varepsilon} \quad R_{S_1} = \frac{r_S(1-\varepsilon)}{\varepsilon} \quad R_{S_2} = \frac{r_S \cdot \tau}{(1-\varepsilon)}$$

Hier ist $\varepsilon$ die Porosität der Polymermatrix, welche wie folgt definiert wird:

$$\varepsilon = \frac{A^P}{A^M}$$

Dabei entspricht $A^P$ der effektiven Fläche der Poren und $A^M$ der totalen Membranfläche. Unter $r_S$ versteht man den spezifischen Widerstand des Membranmaterials. Die Porosität $\varepsilon$ ist definitionsgemäß kleiner als 1. Der Umwegfaktor $\tau$ berücksichtigt den Umstand, daß der Weg bei der Diffusion durch das Polymer bei hoher Porosität des Polymers im allgemeinen länger ist als direkt durch die Polymerschicht und die Flüssigkeit. Damit ergibt sich für die Permeabilität folgende Beziehung:

$$P_i = \frac{1}{\Delta z} \cdot \left[\frac{\varepsilon}{r_T + r_S(1-\varepsilon)} + \frac{(1-\varepsilon)}{r_S \cdot \tau}\right]$$

Für den Fall eines selektiven, trägergebundenen Sauerstofftransportes durch die Flüssigkeit in der Membran, gilt für die Hauptkomponenten der Luft:

a) Stickstoff: $r_T >> r_S$

$$P_{N_2} = \frac{1}{\Delta z} \cdot \left[\frac{(1-\varepsilon)}{r_S \cdot \tau}\right]$$

b) Sauerstoff: $r_T << r_S$

$$P_{O_2} = \frac{1}{\Delta z} \cdot \left[\frac{\varepsilon}{r_S(1-\varepsilon)} + \frac{(1-\varepsilon)}{r_S \cdot \tau}\right]$$

$$P_{O_2} = \frac{1}{\Delta z} \cdot \left[\frac{\varepsilon}{r_S(1-\varepsilon)}\right]$$

Für die Selektivität erhält man schließlich:

$$S_{O_2/N_2} = \frac{P_{O_2}}{P_{N_2}} = \frac{\varepsilon \cdot \tau \cdot r_{S_{N_2}}}{r_{S_{O_2}} (1-\varepsilon)^2}$$

Die Selektivität der beschriebenen, schaumartigen Flüssigmembran wird also zum einen wie bei reinen Polymermembranen durch das Verhältnis der Leitwerte ($1/r_S$) für die zu trennenden Gase bestimmt. Weiterhin wirkt eine Vergrößerung der Porosität fördernd für die Selektivität, wobei festgestellt wurde, daß die Selektivität ab einer Porosität größer als 40% drastisch gesteigert werden kann.

Da sich mit steigender Porosität auch der Diffusionsweg durch das reine Polymer erheblich verlängert, kommt über den Umwegfaktor $\tau$ eine zusätzliche Erhöhung der Selektivität zustande. Setzt man nun eine hohe Selektivität des Flüssigkeitsanteils in der Membran voraus, so kann durch eine hohe Porosität viel Flüssigkeit im Schaum der Membran fixiert werden. Dies hat eine beachtliche Steigerung der Selektivität zur Folge.

Die Porosität der Membran darf jedoch nicht zu hoch gewählt werden, da dies zu einer mechanischen Instabilität der selektiven Trennschicht führt. Da aber bei der Gastrennung mit hohen Drücken als treibende Kraft gearbeitet wird, muß die Membran eine bestimmte Stabilität aufweisen, so daß die Porosität einen bestimmten Grenzwert nicht überschreiten darf. Es muß also ein Kompromiß geschlossen werden zwischen der Anforderung, möglichst viel Flüssigkeit in der Membran fixieren zu können, und einer möglichst hohen mechanischen Stabilität.

Die erfindungsgemäßen Membranen weisen eine ultradünne, nur 0.1 bis 0.5 µm dicke, selektive Oberflächenschicht, eine sog. Toplayer auf, die durch eine 100 bis 300 µm dicke, darunter liegende Stützschicht supportiert wird. Die hochporöse Unterstruktur zeigt gute mechanische Eigenschaften und ist für die gute mechanische Stabilität der erfindungsgemäßen Membran verantwortlich, die dadurch den mechanischen Belastungen durch hohe Drücke, welche auf die dünne Toplayer wirken, standhält.

Weiterhin weist die Unterstruktur der erfindungsgemäßen Membran eine hohe Permeabilität auf, so daß in der porösen Stützschicht nur ein konvektiver Fluß stattfindet (Hagen-Poiseuille-Bereich), der keinen Einfluß auf das Trennvermögen der Membran ausübt, Für den Trenneffekt ist somit nur die dünne, oberste Schicht verantwortlich. Bevorzugt ist es, wenn das Stützmaterial in der Porengröße so gewählt ist, daß der hydrodynamische Strömungswiderstand des Stützmaterials vernachlässigbar gering ist gegenüber dem hydrodynamischen Widerstand der dünnen Membranoberschicht. Es ist jedoch darauf zu achten, daß die nicht abgestützte Membranfläche über einer Pore möglichst klein ist.

Die Trocken-Naß-Phaseninversion wird bevorzugt bei Temperaturen zwischen 15 und 35 °C durchgeführt. Dabei ist stets darauf zu achten, daß die Verarbeitungstemperatur niedriger liegt als der Siedepunkt des leichtflüchtigen Lösungsmittels, da es andernfalls zur Blasenbildung kommt.

Ohne Einschränkung der Allgemeinheit sind Polysulfone, Polyethersulfone, Bisphenol-A Polysulfon, Polyphenylensulfone, Polyimide, Polyetherimide, Polyphenylenoxide, Polycarbonate, Polyestercarbonate, Celluloseacetate, Polyurethane, Polydimethylsiloxan oder Polyetherblockamide geeignete und bevorzugte Polymere zur Herstellung der erfindungsgemäßen Membranen.

Ohne Einschränkung der Allgemeinheit sind geeignete und bevorzugte schwerflüchtige Lösungsmittel zur Herstellung der erfindungsgemäßen Membranen NMP, DMAc, DMF oder DMSO, geeignete und bevorzugte leichtflüchtige Lösungsmittel sind Methylenchlorid, Chloroform, Aceton, Dioxan oder THF.

Bevorzugte Fällmittel sind Wasser, Ethanol oder Methanol, wobei Wasser ganz besonders bevorzugt ist.

Zur Herstellung von erfindungsgemäßen Fest-Carrier-Membranen werden bevorzugt Polymerlösungen eingesetzt, die zu

a) 10 bis 20 Gew.-% aus einem organischen Polymer, zu
b) 40 bis 50 Gew.-% aus einem leichtflüchtigen Lösungsmittel, zu
c) 25 bis 35 Gew.-% aus einem schwerflüchtigen Lösungsmittel, zu
d) 2 bis 10 Gew.-% aus einer oder mehreren stoffspezifischen Trägersubstanzen, und zu
e) 5 bis 10 Gew.-% aus einem Nichtlösungsmittels (Fällmittel) bestehen.

Zur Herstellung von erfindungsgemäßen Flüssigmembranen werden bevorzugt Polymerlösungen eingesetzt, die zu

a) 10 bis 20 Gew.-% aus einem organischen Polymer, zu
b) 35 bis 45 Gew.-% aus einem leichtflüchtigen Lösungsmittel, zu
c) 25 bis 30 Gew.-% aus einem schwerflüchtigen Lösungsmittel, und zu
d) 20 bis 40 Gew.-% aus einer 0.02 bis 0.5 molaren Carrier-Lösung bestehen.

Carrier-Membranen nach dem Stand der Technik enthalten nur geringe Mengen an Carrier, da diese bei zu hohen Konzentrationen kristallisieren und damit inaktiv werden. Da Carrier-Kristalle außerdem gut wachsen, induzieren sie

beim Kristallisieren Fehlstellen in der Membran, die Membran erhält Löcher und wird undicht. Dies Verhalten steht dem Bestreben entgegen, eine möglichst große Carrier-Menge in die Membran zu integrieren, um dadurch den Stofftransport durch die Membran verbessern zu können. Ein weiterer Vorteil der erfindungsgemäßen Membranen besteht nun darin, daß diese gegenüber dem Stand der Technik einen wesentlich höheren Carrier-Gehalt aufzunehmen vermögen.

Entsprechend dem jeweiligen Anwendungsfall der erfindungsgemäßen Membranen können eine ganze Reihe stoffspezifischer Trägersubstanzen zur Herstellung der erfindungsgemäßen Membranen eingesetzt werden. Ohne Einschränkung der Allgemeinheit sind dies z.B. Silber(I)-Salze für die Trennung von Alkanen und Alkenen, Kupfer(I)chlorid für die Abtrennung von Kohlenmonoxid aus Verbrennungsgasen, Ammoniumthiocyanate für Abtrennung von Aminen, langkettige Diamine für die Abtrennung von Kohlendioxid aus Erdgas, Trioctylammonium-Salze in o-Nitrophenyloctylether für die Abtrennung von Nitrat aus Trinkwasser, Eisen(II)-Salze für die Abtrennung von Stickstoffmonoxid aus Verbrennungsgasen, Spiro-indolinbenzopyran für die selektive Abtrennung einwertiger Ionen, Kelex 100 für die Abtrennung von $Cu^{2+}$ aus galvanischen Bädern, LIX 64N für Abtrennung $Cu^{2+}/Fe^{2+}$ aus galvanischen Bädern, Alamin 336 für die Abtrennung von Chromat aus galvanischen Bädern, Mangan-cyclopentadienyl für die Stickstoffabtrennung aus Erdgas, basische Lösungsmittel bzw. basische Medien wie Polyvinylamin oder Polyethylenimin für die Abtrennung saurer Gase ($H_2S$, $SO_2$, etc.) oder Sulfonsäuren zur Abtrennung basischer Gase ($NH_3$, etc.).

Ganz besonders sind in diesem Zusammenhang Metallkomplexe als Carrier für Sauerstoff zur Stickstoff/Sauerstoff-Trennung oder zur Sauerstoffanreicherung zu nennen, wobei unter diesen kobalt-organische Verbindungen ganz besonders gut geeignet sind. Geeignete Metallkomplexe als Sauerstoff-Carrier sind der EP 0098157 B1 sowie der EP 0098731 B1 zu entnehmen.

Die erfindungsgemäßen Membranen sind mit den eben genannten Metallkomplexen als Carrier-Substanzen ganz besonders gut zur Stickstoff/Sauerstoff-Trennung bzw. zur Anreicherung von Sauerstoff aus Luft geeignet. Potentielle Anwendungen hierfür sind z.B. allgemeine Verbrennungsprozesse, denn durch die Anreicherung von Sauerstoff lassen sich hier höhere Flammtemperaturen, verbesserte "Wärme-Ausbeuten" und ein geringerer Brennstoff-Verbrauch erzielen. Weitere Anwendungen liegen bei der Kohleverflüssigung, bei der Herstellung von Peroxiden über einen erhöhten $O_2$-Partialdruck, bei Glas- und Metallschmelzverfahren, in der Abwasserbehandlung und in der Medizintechnik.

Wie eingangs schon erwähnt, werden für die Stickstoff/Sauerstoff-Trennung vorwiegend Co-Salen- und Co-Porphin-Verbindungen als Carrier verwendet. In diesen ist das Zentralatom $Co^{2+}$ von fünf Elektronendonatoren koordiniert, die entweder die Liganden oder eine sog. Axialbase darstellen. Anstelle einer Axialbase kann auch ein geeignetes Lösungsmittel diese Funktion übernehmen. Durch Zugabe einer Axialbase zu einem 4-fach koordinierten Co-Komplex wird der " Bindungszustand " des Co-Atoms zur Koordinationszahl 6 erweitert, wobei die 6. Koordinationsstelle anfangs unbesetzt ist und später bei der Sauerstoffübertragung durch den Sauerstoff eingenommen wird. Dabei wird der molekulare Sauerstoff direkt vom $Co^{2+}$-Atom durch partielle Elektronenübertragung gebunden.

Geeignete und bevorzugte Axialbasen für Co-Salen-Derivate sind z.B. 4-Dimethylaminopyridin, 1-Methylimidazol, 4-Cyanopyridin, Perfluortributylamin, Oligo(4-vinylpyridin) oder Oligo(1-vinylimidazol). Bei besonders bevorzugten Ausführungsformen des erfindungsgemäßen Verfahrens werden Axialbasen eingesetzt, die über einen niedrigen Dampfdruck verfügen, wie z.B. 4-Dimethylaminopyridin.

Wie eingangs bereits erwähnt wurde, sind die für die Stickstoff/Sauerstoff-Trennung als Sauerstoff-Carrier eingesetzten Metallkomplexe in hohem Maße oxidationsanfällig. Abbildung 3 zeigt schematisch am Beispiel eines Co-Komplexes die irreversible Sauerstoff-Bindung, d.h. die irreversible Oxidation des mit Sauerstoff beladenen Carriers über die Superoxo- und Peroxo-Form zur Oxo-Form. Diese Oxidationsanfälligkeit der Sauerstoff-Carrier nach dem Stand der Technik macht längere Standzeiten unmöglich.

Überraschenderweise wurden nun in den folgenden kobalt-organischen Verbindungen Sauerstoff-Carrier gefunden, die eine wesentlich verbesserte Stabilität gegenüber molekularem Sauerstoff zeigen, und die damit wesentlich längere Standzeiten z.B. bei der Stickstoff/Sauerstoff-Trennung aufweisen.

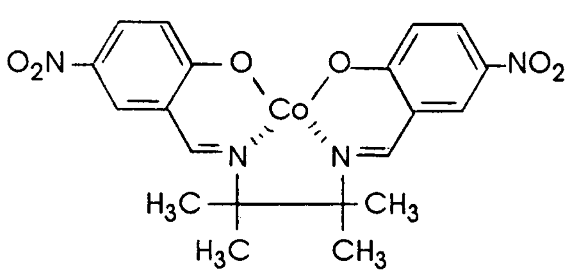 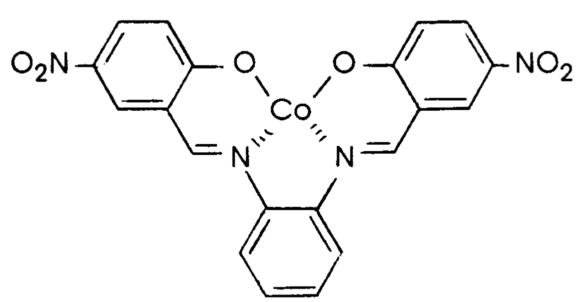

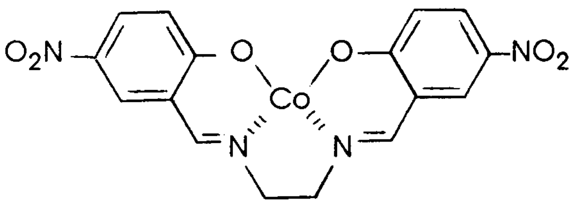 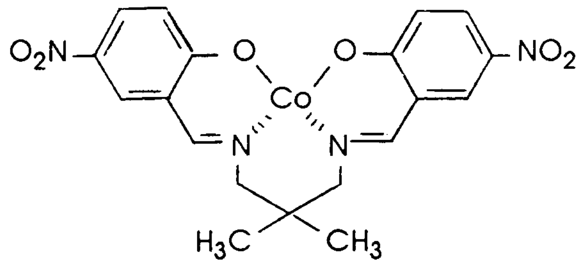

Überraschenderweise wurden damit Co-Verbindungen gefunden, bei denen das Redox-Potential des Zentralatoms zwar ausreicht, um Elektronen partiell auf Sauerstoff zu übertragen, aber nicht, um den Sauerstoff vollständig zu einem Superoxid oder gar Peroxid zu reduzieren. Damit erfüllen diese Verbindungen die Anforderungen, die an einen optimalen Sauerstoff-Carrier gestellt werden, nämlich reversible Sauerstoff-Bindung, chemische Stabilität, auch gegenüber Sauerstoff, hohe Geschwindigkeit der Sauerstoff-Aufnahme und -Abgabe, sowie Mobilität der Komplexe Im flüssigen und polymeren Teil der Membran.

Es wird angenommen, daß durch den Einbau von zwei Nitro-Gruppen (elektronischer Effekt), jeweils in para-Stellung zum Sauerstoff-Liganden, das Reduktionspotential des Kobalts so verändert wird, daß der Co-Komplex gegenüber Oxidationen stabilisiert ist. Desweiteren wird angenommen, daß durch die Modifizierung der N-N-Brücke (sterischer Effekt) eine zusätzliche Stabilisierung erfolgt.

Die Abbildungen 4 bis 6 zeigen bevorzugte, sowie mit einer Axialbase versehene und mit Sauerstoff beladene Co-Salen-Verbindungen.

Bei der Herstellung der erfindungsgemäßen Membranen unter Verwendung von Co-Salen-Verbindungen als Sauerstoff-Carrier muß darauf geachtet werden, daß isolierte und nicht mit Sauerstoff beladene Carrier-Moleküle in die Membran eingebracht werden. Dies kann z.B. durch Arbeiten unter Schutzgas bewerkstelligt werden.

Die erfindungsgemäßen Carrier-Membranen können in Abhängigkeit der verwendeten Carrier-Substanzen für die unterschiedlichsten Trennprozesse eingesetzt werden. Ohne Einschränkung der Allgemeinheit sind diese:

- Anreicherung von Sauerstoff aus Luft; erleichterter Transport von Sauerstoff-Co-balt(II)-Salene
- Trennung von Alkanen und Alkenen; erleichterter Transport von z.B. Ethylen, Propylen durch reversible Bindung an Ag(I)-Salze
- Abtrennung basischer Gase wie z.B. Amine aus Synthesegasen; reversible Bindung des Amins an Ammoniumthiocyanate
- Abtrennung von Stickstoffmonoxid aus Verbrennungsgasen durch reversible Bindung an Fe(II)-Salze
- Abtrennung von Kohlenmonoxid aus Verbrennungsgasen durch reversible Bindung an Cu(I)Cl
- Abtrennung von Kohlendioxid aus Erdgas durch reversible Bindung an langkettige Diamine
- Abtrennung von Nitrat aus Trinkwasser durch reversible Bindung an Trioctylammonium-Salze in o-Nitrophenyloctylether
- Selektive Abtrennung 1-wertiger Ionen über ein gelöstes Spiro-indolinbenzopyran
- Abtrennung von Schwermetallen aus galvanischen Bädern durch reversible Komplexierung, wie z.B.
  $Cu^{++}$ durch Kelex 100
  $Cu^{++}$ / $Fe^{++}$ mittels LIX 64N
  Chromat mittels Alamin 336
- Selektive Abtrennung von Stickstoff aus Erdgas zur Brennwertkonditionierung mittels reversibler Bindung an Mn-cyclopentadienyl-Verbindungen
- Abtrennung von sauren Gasen, wie z.B. $H_2S$, aus Erdgas
- Abtrennung von Schwefeldioxid aus Rauchgasen mittels reversibler Bindung an basische Verbindungen

Anhand von Ausführungsbeispielen werden die erfindungsgemäßen Membranen näher erläutert.

**Beispiel 1:**

Integral asymmetrische Fest-Carrier-Membran

Rezeptur: 100 g Dichlormethan
70 g N-Methylpyrrolidon
30 g Polysulfon

5 g Co(5-$NO_2$-Saltmen) • DMAP
≈ 8 g 1-Propanol

| | |
|---|---|
| Erzwungene Konvektion: | 20 Sekunden |
| Freie Konvektion : | 15 Sekunden |

Durchführung:

Der Carrier Co(5-$NO_2$-Saltmen) • DMAP wird in 70 g N-Methylpyrrolidon in der Hitze unter Argon und in desorbiertem Zustand (Sauerstoff ist desorbiert) gelöst. Nun wird bei Raumtemperatur über 0.45 µm filtriert und die Lösung im Vakuum entgast. Das Polysulfon wird in Methylenchlorid gelöst, über 2 µm filtriert und im Vakuum entgast. In diese rotbraune Lösung wird nun unter langsamen Rühren 1-Propanol (5 - 7 ml) eingebracht, bis sich eine leichte Opaleszenz einstellt. Nach Rakeln auf Glas (200 µm) wird mit Stickstoff das leichtflüchtige Lösungsmittel abgeblasen, die Glasplatte unter Stickstoff kurz konditioniert und schließlich in Wasser koaguliert. Nach Ablösen von der Glasplatte wird kurz in Ethanol gespült und im Stickstoffstrom abgelüftet. Die Membran weist im statistischen Mittel eine Selektivität von $S_{(O2/N2)}$ = 23 bei 30 °C und einen normalisierten Fluß von etwa 23 000 Ba/cm auf. Im REM ist ein sich nach oben verdichteter Schaum und eine porenfreie, dichte Haut an der Oberseite zu erkennen.

**Beispiel 2:**

Mikroinkapsulierte asymmetrische Flüssigmembran

Rezeptur: 100g Dichlormethan
70g N-Methylpyrrolidon
30 g Polysulfon
30 g Carrier-Lösung (.7 m Co(5-$NO_2$-Saltmen) • DMAP in Diethylengly coldibutylether)

| | |
|---|---|
| Erzwungene Konvektion: | 35 Sekunden |
| Freie Konvektion | 15 Sekunden |

Durchführung:

Der Carrier [Co(5-$NO_2$-Saltmen) • DMAP] wird in 30 g Diethylenglycoldibutylether und 30g N-Methylpyrrolidon in der Hitze unter Argon und in desorbiertem Zustand gelöst. Nun wird bei Raumtemperatur über 0.45 µm filtriert und die Lösung im Vakuum entgast. Das Polysulfon wird in einem Gemisch aus Methylenchlorid und N-Methylpyrrolidon gelöst, über 2 µm filtriert und im Vakuum entgast. Nun werden die beiden Lösungen vermischt und gemeinsam mit Hilfe eines Ultraturax emulgiert. In diese tiefrote Lösung wird nun unter langsamen Rühren 1-Propanol (5 - 7 ml) eingebracht, bis sich eine leichte Opaleszenz einstellt. Nach Rakeln auf Glas (200 µm) wird mit Stickstoff das leichtflüchtige Lösungsmittel abgeblasen, die Glasplatte unter Stickstoff kurz konditioniert und schließlich in Wasser koaguliert. Nach Ablösen von der Glasplatte wird kurz in Ethanol gespült und im Stickstoffstrom abgelüftet.

| | | |
|---|---|---|
| Versuchsbedingungen: | Temperatur | 20 °C |
| | Feeddruck | 1.5 bar |
| | Permeatdruck | Atmosphärendruck (1 bar) |
| | Membranfläche | 63.62 $cm^2$ |
| | Stage-Cut | 0.1 |
| | Druckverhältnis | 0.14 |

Carrierhaltige Membranen Zeigen nach Phaseninversion im Wasser eine reale Selektivität von $S^{real}$ = 16.1. Da die reale Selektivität die eines carrierfreien asymmetrischen Polysulfonfilms deutlich überschreitet, wird ein trägergebundener Sauerstoff-Transport angenommen.

**Ergebnis:**

| mikroinkapsulierte Flüssigmembran, Co(5-$NO_2$-Saltmen)/DEGBE | | | |
|---|---|---|---|
| Membran | Fluß $O_2$ [ml/min] | P/z$O_2$ [Ba/cm] | reale Selektivität |
| 1 | 1.71 | 40482 | 19.7 |
| 2 | 2.48 | 58711 | 13.2 |
| 3 | 2.22 | 52556 | 15.9 |
| 4 | 2.11 | 49952 | 15.6 |
| analoge, carrierfreie integral asymmetrische Gastrennmembran | | | |
| Membran | Fluß $O_2$ [ml/min] | P/z$O_2$ [Ba/cm] | reale Selektivität |
| 1 | 2.29 | 54212 | 6.58 |
| 2 | 2.13 | 50424 | 6.91 |
| 3 | 2.63 | 62261 | 6.27 |
| 4 | 1.99 | 47110 | 6.92 |

**Patentansprüche**

1. Stoffspezifische Trägersubstanzen (Carrier) enthaltende Polymermembran, erhältlich durch Trocken-Naß-Phaseninversion, indem man aus einer homogenen, flüssigen Polymerlösung, bestehend aus

   a) 8 bis 35 Gew.-% eines organischen Polymers,
   b) 20 bis 60 Gew.-% eines leichtflüchtigen Lösungsmittels,
   c) 15 bis 80 Gew.-% eines schwerflüchtigen Lösungsmittels,

d) 1 bis 20 Gew.-% einer oder mehrerer stoffspezifischer Trägersubstanzen, bzw. 10 bis 60 Gew.-% einer 0.02 bis 0.5 molaren Lösung der stoffspezifischen Trägersubstanzen in einem schwerflüchtigen, mit dem Fällungsmittel für die Phaseninversion nicht mischbaren Lösungsmittel, und gegebenenfalls

e) 5 bis 15 Gew.-% eines Nichtlösungsmittels (Fällmittel),

einen Polymerfilm fertigt, daraus das leichtflüchtige Lösungsmittel entfernt und den resultierenden Polymerfilm mittels eines Fällmittels einer Phaseninversion unterzieht.

2. Polymermembran nach Anspruch 1, **dadurch gekennzeichnet**, daß das Polymer bevorzugt ein Polysulfon, Bisphenol-A Polysulfon, ein Polydimethylsiloxan, ein Polyethersulfon, ein Polyphenylsulfon, ein Polyimid, ein Polyetherimid, ein Polyphenylenoxid, ein Polycarbonat, ein Polyestercarbonat, ein Celluloseacetat, ein Polyurethan oder ein Polyetherblockamid ist.

3. Polymermembran nach Anspruch 1 oder 2, **dadurch gekennzeichnet**, daß die stoffspezifischen Trägersubstanzen Metallkomplexe sind.

4. Polymermembran nach Anspruch 3, **dadurch gekennzeichnet**, daß die Metallkomplexe Kobalt-organische, gegebenenfalls mit einer Axialbase koordinierte Verbindungen sind.

5. Polymermembran nach Anspruch 4), **dadurch gekennzeichnet**, daß die Kobalt-organische Verbindung bevorzugt

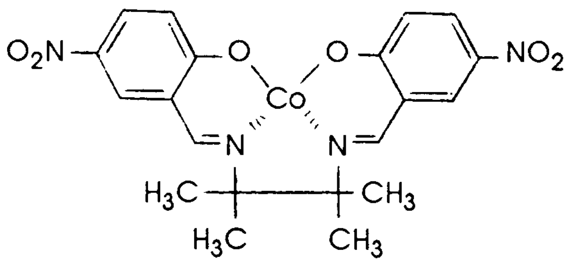

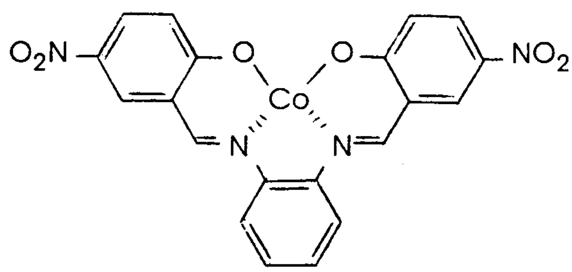

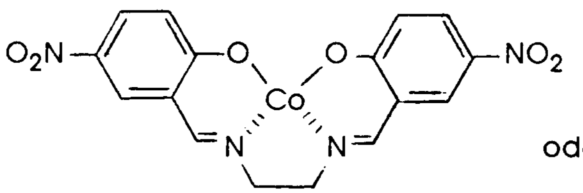

oder

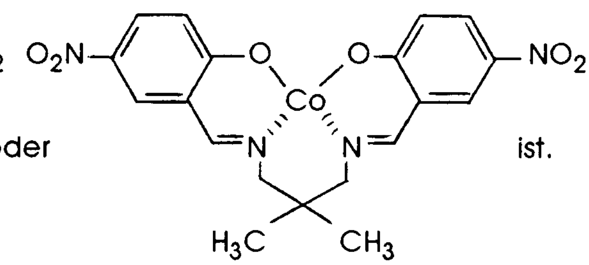

ist.

6. Polymermembran noch Anspruch 4 oder 5, **dadurch gekennzeichnet**, daß die Axial-Base bevorzugt 4-Dimethylaminopyridin, 1-Methylimidazol, 4-Cyanopyridin, Perfluortributylamin, Oligo(4-vinylpyridin) oder Oligo(1-vinylimidazol) ist.

7. Polymermembran nach Anspruch 1 oder 2, **dadurch gekennzeichnet**, daß die stoffspezifischen Trägersubstanzen Silber(I)-Salze, Kupfer(I)-chlorid, Mangancyclopentadienyl-Verbindungen, Polyvinylamin, Polyethylenimin, Ammoniumthiocyanate, Eisen(II)-Salze, langkettige Diamine, Trioctylammonium-Salze in o-Nitrophenyloctylether, Spiro-indolinbenzopyran, Polyacrylsäure oder Sulfonsäuren sind.

8. Verfahren zur Herstellung von stoffspezifische Trögersubstanzen (Carrier) enthaltende Polymermembranen nach einem oder mehreren der Ansprüche 1 bis 7 durch Trocken-Naß-Phaseninversion, **dadurch gekennzeichnet**, daß man aus einer homogenen, flüssigen Polymerlösung, welche aus

a) 8 bis 35 Gew.-% eines organischen Polymers,
b) 20 bis 60 Gew.-% eines leichtflüchtigen Lösungsmittels,
c) 15 bis 80 Gew.-% eines schwerflüchtigen Lösungsmittels,

d) 1 bis 20 Gew.-% einer oder mehrerer stoffspezifischer Trägersubstanzen, bzw. 10 bis 60 Gew.-% einer 0.02 bis 0.5 molaren Lösung der stoffspezifischen Trägersubstanzen in einem schwerflüchtigen, mit dem Fällungsmittel für die Phoseninversion nicht-mischbaren Lösungsmittel, und gegebenenfalls

e) 5 bis 15 Gew.-% eines Nichtlösungsmittels (Fällmittel),besteht,

einen Polymerfilm fertigt, daraus das leichtflüchtige Lösungsmittel entfernt und den resultierenden Polymerfilm mittels eines Fällmittels einer Phaseninversion unterzieht.

**9.** Verfahren nach Anspruch 8), **dadurch gekennzeichnet**, daß man eine Polymerlösung verwendet, die bevorzugt aus

a) 10 bis 20 Gew.-% eines organischen Polymers,
b) 40 bis 50 Gew.-% eines leichtflüchtigen Lösungsmittels,
c) 25 bis 35 Gew.-% eines schwerflüchtigen Lösungsmittels,
d) 2 bis 10 Gew.-% einer oder mehrerer stoffspezifischer Trögersubstanzen, und
e) 5 bis 10 Gew.-% eines Nichtlösungsmittels (Fällmittel) besteht.

**10.** Verfahren nach Anspruch 8), **dadurch gekennzeichnet**, daß man eine Polymerlösung verwendet, die bevorzugt aus

a) 10 bis 20 Gew.-% eines organischen Polymers,
b) 35 bis 45 Gew.-% eines leichtflüchtigen Lösungsmittels,
c) 25 bis 30 Gew.-% eines schwerflüchtigen Lösungsmittels, und
d) 20 bis 40 Gew.-% einer 0.02 bis 0.5 molaren Carrier-Lösung besteht.

**11.** Verfahren nach Anspruch 8, 9 oder 10, **dadurch gekennzeichnet**, daß man als Polymer bevorzugt ein Polysulfon, Bisphenol-A Polysulfon, ein Polydimethylsiloxan, ein Polyethersulfon, ein Polyphenylsulfon, ein Polyimid, ein Polyetherimid, ein Polyphenylenoxid, ein Polycarbonat, ein Polyestercarbonat, ein Celluloseacetat, ein Polyurethan oder ein Polyetherblockamid verwendet.

**12.** Verfahren nach einem oder mehreren der Ansprüche 8 bis 11, **dadurch gekennzeichnet**, daß man als stoffspezifische Trägersubstanzen Metallkomplexe verwendet.

**13.** Verfahren nach Anspruch 12, **dadurch gekennzeichnet**, daß man als Metallkomplexe kobalt-organische, gegebenenfalls mit einer Axialbase koordinierte Verbindungen verwendet.

**14.** Verfahren nach Anspruch 13), **dadurch gekennzeichne**, daß man als kobalt-organische Verbindung bevorzugt

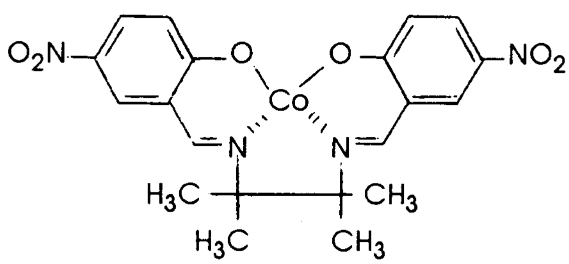
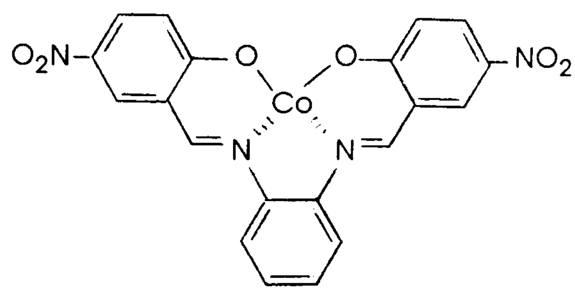
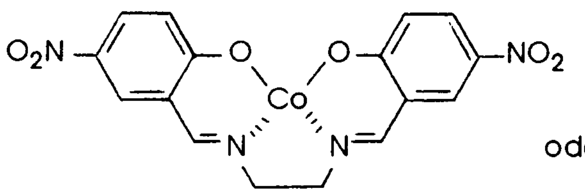
oder
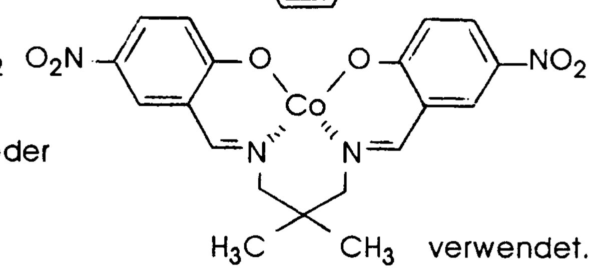
verwendet.

**15.** Verfahren nach Anspruch 13 oder 14, **dadurch gekennzeichnet**, daß man als Axial-Base bevorzugt 4-Dimethylaminopyridin, 1-Methyllmldazol, 4-Cyanopyridin, Perfluortributylamin, Oligo(4-vinylpyridin) oder Oligo(1-vinylimidazol) verwendet.

**16.** Verfahren nach einem oder mehreren der Ansprüche 8 bis 11, **dadurch gekennzeichnet**, daß man als stoffspezifische Trägersubstanzen Silber-(I)-Salze, Kupfer-(I)-chlorid, Mangan-cyclopentadienyl-Verbindungen, Polyvinylamin, Polyethylenimin, Ammoniumthiocyanate, Eisen-(II)-Salze, langkettige Diamine, Trioctylammonium-Salze in o-Nitrophenyloctylether, Spiro-indolinbenzopyran, Polyacrylsäure oder Sulfonsäuren verwendet.

**17.** Verwendung der Membranen nach einem oder mehreren der Ansprüche 1 bis 7 zur selektiven Trennung flüssiger oder gasförmiger, molekularer Gemische.

**18.** Verwendung nach Anspruch 17 zur Anreicherung von Sauerstoff aus Luft, zur Trennung von Alkanen und Alkenen, zur Abtrennung basischer Gase aus Synthesegasen, zur Abtrennung von Stickstoffmonoxid aus Verbrennungsgasen, zur Abtrennung von Kohlenmonoxid aus Verbrennungsgasen, zur Abtrennung von Kohlendioxid aus Erdgas, zur Abtrennung von Nitrat aus Trinkwasser, zur selektiven Abtrennung 1-wertiger Ionen, zur Abtrennung von Schwermetallen aus galvanischen Bädern, zur Abtrennung von Stickstoff aus Erdgas, zur Abtrennung saurer Gase aus Erdgas und zur Abtrennung von Schwefeldioxid aus Rauchgasen.

## Claims

**1.** Polymer membrane containing material-specific carriers which may he obtained by dry-wet phase inversion by producing a polymer film from a homogeneous liquid polymer solution composed of:

a) 8 to 35% by weight of an organic polymer,
b) 20 to 60% by weight of a readily volatile solvent,
c) 15 to 80% by weight of a difficult to volatilise solvent,
d) 1 to 20% by weight of one or several material-specific carriers, or 10 to 60% by weight of a 0.02 to 0.5 molar solution of the material-specific carriers in a solvent, which is difficult to volatilise and is not mixable with the precipitant for the phase inversion, and optionally
e) 5 to 15% by weight of a non-solvent (precipitant),

by removing the readily volatile solvent therefrom and subjecting the resulting polymer film to a phase inversion by means of a precipitant.

**2.** Polymer membrane according to Claim 1, characterised in that the polymer is preferably a polysulphone, bisphenol A polysulphone, a polydimethyl siloxane, a polyether sulphone, a polyphenyl sulphone, a polyimide, a polyether imide, a polyphenylene oxide, a polycarbonate, a polyester carbonate, a cellulose acetate, a polyurethane or a polyether block amide.

**3.** Polymer membrane according to Claim 1 or 2, characterised in that the material-specific carriers are metal complexes.

**4.** Polymer membrane according to Claim 3, characterised in that the metallic complexes are cobalt organic compounds, optionally coordinated with an axial base.

5. Polymer membrane according to Claim 4, characterised in that the cobalt organic compound is preferably

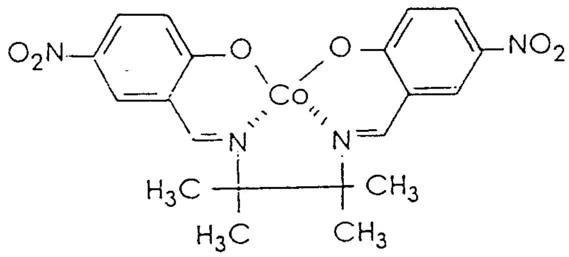
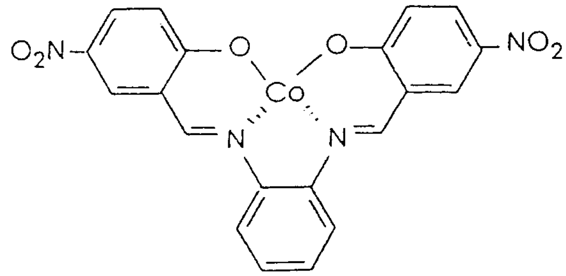
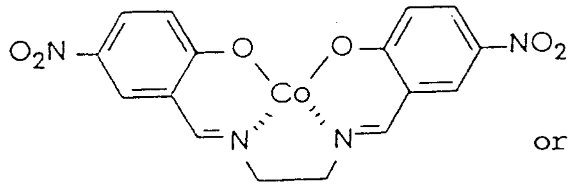

or

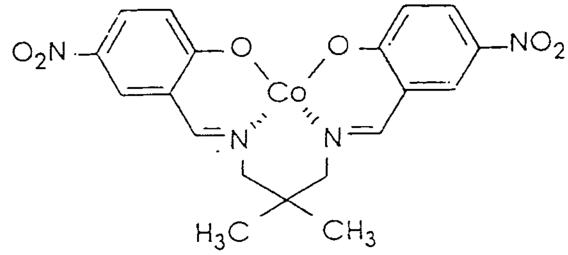

6. Polymer membrane according to Claim 4 or 5, characterised in that the axial base is preferably 4-dimethylaminopyridine, 1-methylimidazole, 4-cyanopyridine, perfluorotributyl amine, oligo(4-vinyl pyridine) or oligo(1-vinyl imidazole).

7. Polymer membrane according to Claim 1 or 2, characterised in that the material-specific carriers are silver(I) salts, copper(I) chloride, manganese cyclopentadienyl compounds, polyvinyl amine, polyethylene imine, ammonium thiocyanate, iron(II) salts, long-chain diamines, trioctyl ammonium salts in o-nitrophenyl octyl ether, spiro-indolinbenzopyrane, polyacrylic acid or sulphonic acids.

8. Process for the production of polymer membranes containing material-specific carriers according to one or more of Claims 1 to 7 by dry-wet phase inversion, characterised in that a polymer film is produced from a homogeneous liquid polymer solution which is composed of:

a) 8 to 35% by weight of an organic polymer,
b) 20 to 60% by weight of a readily volatile solvent,
c) 15 to 80% by weight of a difficult to volatilise solvent,
d) 1 to 20% by weight of one or several material-specific carriers, or 10 to 60% by weight of a 0.02 to 0.5 molar solution of the material-specific carriers in a solvent which is difficult to volatilise and is not mixable with the precipitant for the phase inversion, and optionally
e) 5 to 15% by weight of a non-solvent (precipitant),

the readily volatile solvent is removed therefrom and the resulting polymer film is subjected to a phase inversion by means of a precipitant.

9. Process according to Claim 8, characterised in that the polymer membrane used is preferably composed of:

a) 10 to 20% by weight of an organic polymer,
b) 40 to 50% by weight of a readily volatile solvent,
c) 25 to 35% by weight of a difficult to volatilise solvent,
d) 2 to 10% by weight of one or several material-specific carriers, and
e) 5 to 10% by weight of a non-solvent (precipitant).

10. Process according to Claim 8, characterised in that the polymer membrane used is preferably composed of:

a) 10 to 20% by weight of an organic polymer,
b) 35 to 45% by weight of a readily volatile solvent,

c) 25 to 30% by weight of a difficult to volatilise solvent, and

d) 20 to 40% by weight of a 0.02 to 0.5 molar solution.

**11.** Process according to Claim 8, 9 or 10, characterised in that the polymer used is preferably a polysulphone, bisphenol A polysulphone, a polydimethyl siloxane, a polyether sulphone, a polyphenyl sulphone, a polyimide, a polyether imide, a polyphenylene oxide, a polycarbonate, a polyester carbonate, a cellulose acetate, a polyurethane or a polyether block amide.

**12.** Process according to one or more of Claims 8 to 11, characterised in that the material-specific carriers used are metal complexes.

**13.** Process according to Claim 12, characterised in that the metallic complexes used are cobalt organic compounds, optionally coordinated with an axial base.

**14.** Polymer membrane according to Claim 13, characterised in that the cobalt organic compound used is preferably

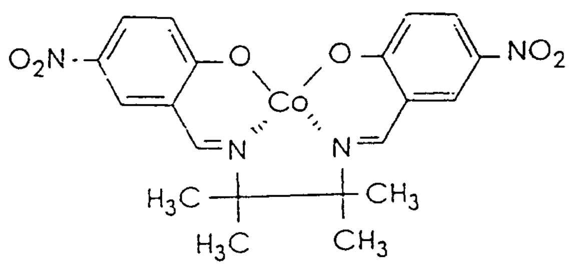

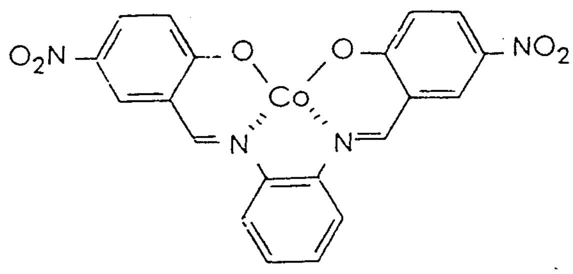

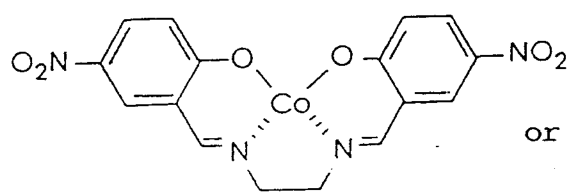

or

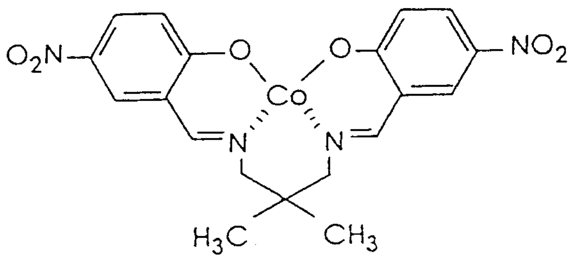

**15.** Process according to Claim 13 or 14, characterised in that the axial base used is preferably 4-dimethylaminopyridine, 1-methylimidazole, 4-cyanopyridine, perfluorotributyl amine, oligo(4-vinyl pyridine) or oligo(1-vinyl imidazole).

**16.** Process according to one or more of Claims 8 to 11, characterised in that the material-specific carriers used are silver(I) salts, copper(I) chloride, manganese cyclopentadienyl compounds, polyvinyl amine, polyethylene imine, ammonium thiocyanate, iron(II) salts, long-chain diamines, trioctyl ammonium salts in o-nitrophenyl octyl ether, spiro-indolinbenzopyrane, polyacrylic acid or sulphonic acids.

**17.** Utilization of the membranes according to one or more of Claims 1 to 7 for the selective separation of liquid or gaseous molecular mixtures.

**18.** Utilisation according to Claim 17 for the enrichment of oxygen from air, for the separation of alkanes and alkenes, for the dissociation of basic gases from synthesis gases, for the dissociation of nitrogen monoxide from combustion gases, for the dissociation of carbon monoxide from combustion gases, for the dissociation of carbon dioxide from natural gas, for the separation of nitrate from drinking water, for the selective dissociation of monovalent ions, for the dissociation of heavy metals from electroplating baths, for the dissociation of nitrogen from natural gas, for the dissociation of acid gases from natural gas and for the dissociation of sulphur dioxide from flue gases.

## Revendications

1. Membrane de polymère contenant des substances support (de substance « carrier ») spécifiques à la matière qu'on obtient par inversion de phases sèche-humide, en fabriquant un film de polymère à partir d'une solution de polymère liquide homogène composée de :

   a) de 8 à 35 % en poids d'un polymères organique,
   b) de 20 à 60 % en poids d'un solvant facilement volatil,
   c) de 15 à 80 % en poids d'un solvant difficilement volatil,
   d) de 1 à 20 % en poids d'une ou plusieurs substances support spécifique à la matière ou 10 à 60 % en poids d'une solution 0,02 à 0,5 molaire des substances support spécifiques à la matrice dans un solvant difficilement volatil, on miscible avec l'agent de précipitation pour l'inversion de phases, et éventuellement,
   e) de 5 à 15 % en poids d'un non solvant (agent de précipitation),

   en éliminant de cela le solvant facilement volatil et en soumettant le film polymère résultant à une inversion de phases au moyen d'un agent de précipitation.

2. Membrane de polymère selon la revendication 1, caractérisée en ce que le polymère est de préférence un polysulfone, un bisphénol-A polysulfone, un polydiméthylsiloxane, un polyéthersulfone, un polyphénylsulfone, un polyimide, un polyétherimide, un polyphénylène-oxyde, un polycarbonate, un polyestercarbonate, un acétate de cellulose, un polyuréthanne, un amide de polyéther en bloc.

3. Membrane de polymère selon les revendications 1 ou 2, caractérisée en ce que les substances support spécifiques à la matière sont des complexes métalliques.

4. Membrane de polymère selon la revendication 3, caractérisée en ce que les complexes métalliques sont des composés organiques de cobalt, éventuellement coordonnés avec une base axiale.

5. Membrane de polymère selon la revendication 4, caractérisée en ce qu'on préfère le composé organique du cobalt :

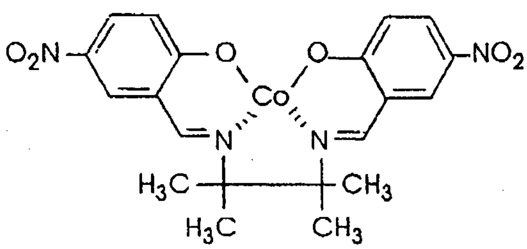
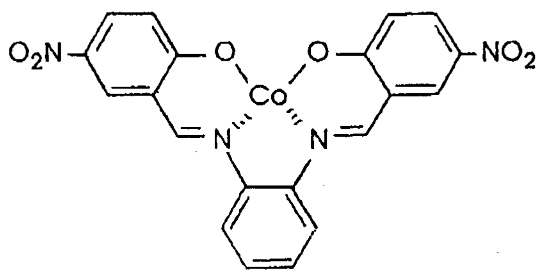

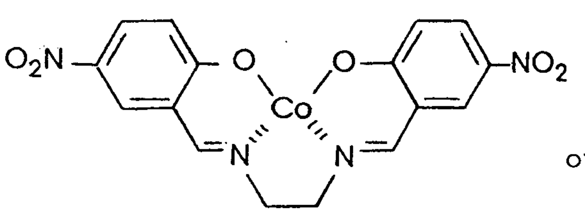
ou
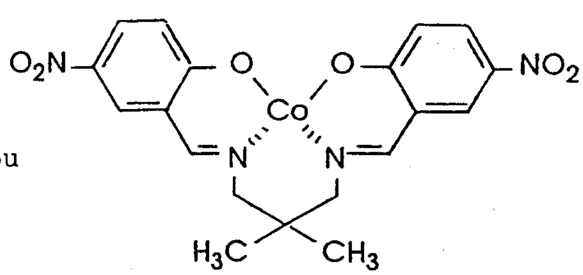

6. Membrane de polymère selon les revendications 4 ou 5, caractérisée en ce qu'on préfère la base axiale 4-diméthylaminopyridine, 1-méthylimidazol, 4-cyanopyridine, perfluorotributylamine, oligo(4-vinylpyridine) ou oligo(1-imidazol).

7. Membrane de polymère selon les revendications 1 ou 2, caractérisée en ce que les substances support spécifiques de matière sont des sels d'argent (I), du chlorure de cuivre (I), des composés de cyclopentadiényl de manganèse, de l'amine de polyvinyle, de l'imine de polyéthylène, du thiocyanate d'ammonium, des sels de fer (II), des diamines à longues chaînes, des sels de trioéthylammonium dans O-nitrophényloctyléther, du spiro-indolinbenzopyrane, de l'acide polyacrylique ou des acides sulfoniques.

8. Procédé de fabrication de membranes polymères contenant des substances support (« carrier ») spécifiques à la matière selon une ou plusieurs des revendications 1 à 7 par inversion de phases sèche-humide, caractérisé en ce qu'on produit un film de polymère à partir d'une solution de polymère liquide homogène, qui se compose de :

   a) de 8 à 35 % en poids d'un polymères organique,
   b) de 20 à 60 % en poids d'un solvant facilement volatil,
   c) de 15 à 80 % en poids d'un solvant difficilement volatil,
   d) de 1 à 20 % en poids d'une ou plusieurs substances support spécifique de matière ou 10 à 60 % en poids d'une solution 0,02 à 0,5 molaire des substances support spécifiques de matrice dans un solvant difficilement volatil, on miscible avec l'agent de précipitation pour l'inversion de phases, et éventuellement,
   e) de 5 à 15 % en poids d'un non solvant (agent de précipitation),

   on élimine de cela le solvant facilement volatil et on soumet le film polymère résultant au moyen d'un agent de précipitation à une inversion de phases.

9. Procédé selon la revendication 8, caractérisé en ce qu'on utilise une solution de polymère, qui se compose de préférence de :

   a) de 10 à 20 % en poids d'un polymères organique,
   b) de 40 à 50 % en poids d'un solvant facilement volatil,
   c) de 25 à 35 % en poids d'un solvant difficilement volatil,
   d) de 2 à 10 % en poids d'une ou plusieurs substances support de matière et,
   e) de 5 à 10 % en poids d'un agent non solvant (agent de précipitation).

10. Procédé selon la revendication 8, caractérisé en ce qu'on utilise une solution de polymère, qui se compose de préférence de :

   a) de 10 à 20 % en poids d'un polymère organique,
   b) de 35 à 45 % en poids d'un solvant facilement volatil,
   c) de 25 à 30 % en poids d'un solvant difficilement volatil, et
   d) de 20 à 40 % en poids d'une solution molaire de substance «carrier» de 0,02 à 0,5 .

11. Procédé selon les revendications 8, 9 ou 10, caractérisé en ce qu'on utilise comme polymère de préférence un polysulfone, du bisphénol-A polysulfone, un polydiméthylsiloxane, un polyéthersulfone, un polyphénylsulfone, un polyimide, un polyétherimide, un polyphénylèneoxyde, un polycarbonate, un polyester carbonate, un acétate de cellulose, un polyuréthanne ou un amide de polyéther en bloc.

12. Procédé selon une ou plusieurs des revendications 8 à 11, caractérisé en ce qu'on utilise comme substances support spécifiques de matière des complexes métalliques.

13. Procédé selon la revendication 12, caractérisé en ce qu'on utilise comme complexes métalliques des composés organiques du cobalt, coordonnés éventuellement par une base axiale.

14. Procédé selon la revendication 13, caractérisé en ce qu'on utilise de préférence comme composé organique du cobalt :

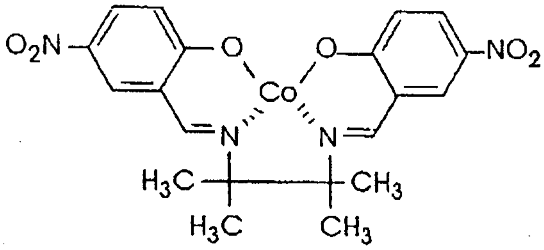

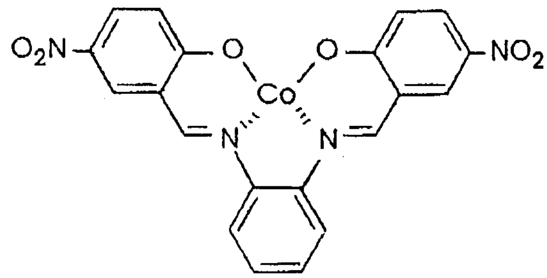

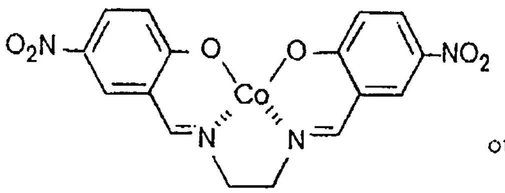

ou

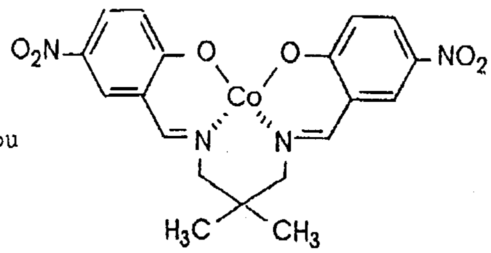

**15.** Procédé selon les revendications 13 ou 14, caractérisé en ce qu'on utilise comme base axiale de préférence la 4-diméthylaminopyridine, le 1-méthylimidazol, le 4-cyanopyridine, la perfluortributylamine, l'oligo(4-vinylpyridine) ou oligo(1-vinylimidazol).

**16.** Procédé selon une ou plusieurs des revendications 8 à 11, caractérisé en ce qu'on utilise comme substances support spécifiques de matière des sels d'argent (I), du chlorure de cuivre (I), des composés de cyclopentadienyl de manganèse, de l'amine de polyvinyle, de l'imine de polyéthylène, des thiocyanates d'ammonium, des sels de fer (II), des diamines à longue chaîne, des sels de triactylammonium dans 0-nitrophényloctyléther, du spiroindolinbenzopyrane, de l'acide polyacrylique ou des acides sulfoniques.

**17.** Utilisation des membranes selon une ou plusieurs des revendications 1 à 7 pour la séparation sélective de mélanges moléculaires liquides ou gazeux.

**18.** Utilisation selon la revendication 17 pour l'enrichissement de l'oxygène de l'air, pour la séparation d'alcanes ou d'alcènes, pour la séparation de gaz basiques de gaz de synthèse, pour la séparation de monoxydes d'azote des gaz de combustion, pour la séparation de monoxyde de carbone des gaz de combustion, pour la séparation de gaz carbonique du gaz naturel, pour la séparation de nitrate de l'eau potable, pour la séparation sélective d'ions monovalents, pour la séparation de métaux lourds de bains galvanoplastiques, pour la séparation d'azote du gaz naturel, pour la séparation de gaz acides du gaz naturel et pour la séparation d'anhydride sulfureux de gaz de fumée.

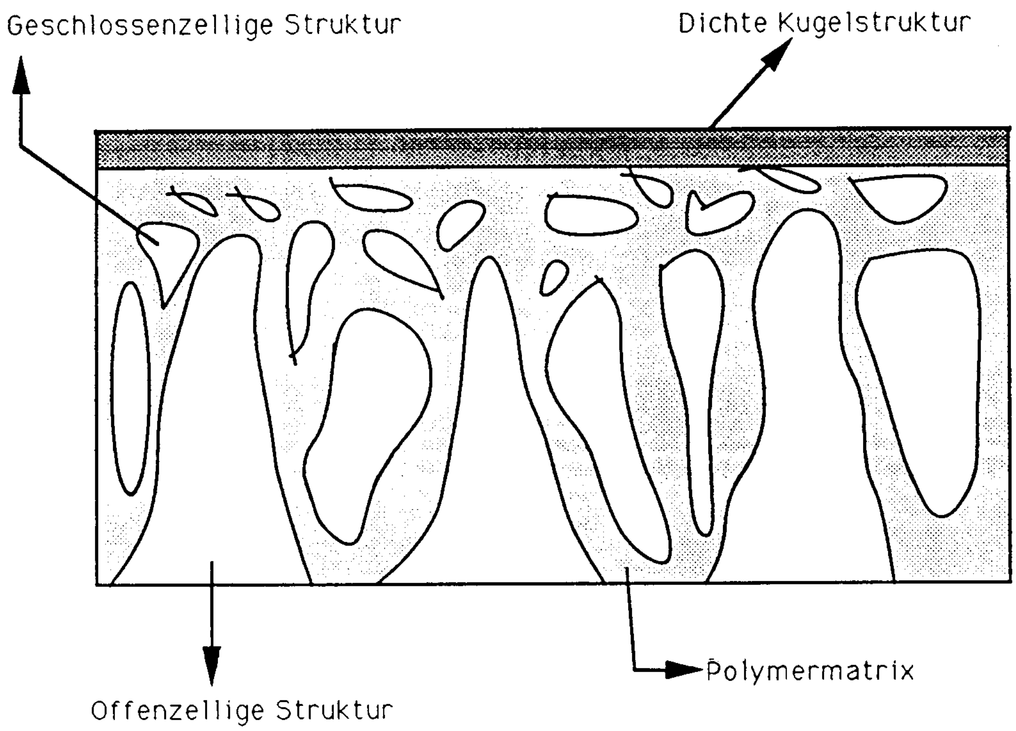
Geschlossenzellige Struktur
Dichte Kugelstruktur
Offenzellige Struktur
Polymermatrix

Abb. 1.

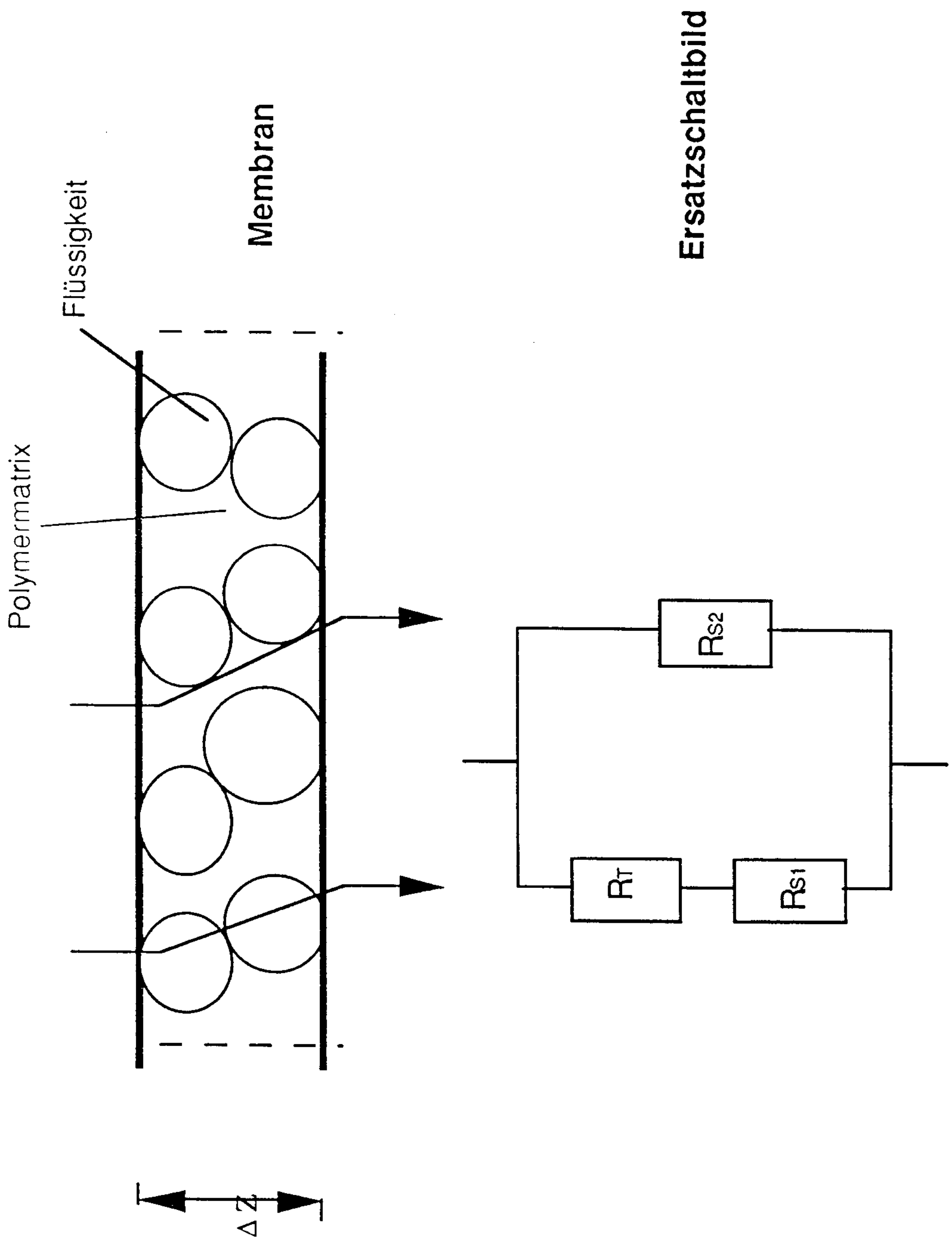
Membran
Flüssigkeit
Polymermatrix
Δ Z
Ersatzschaltbild
$R_{S2}$
$R_T$
$R_{S1}$

Abb.2.

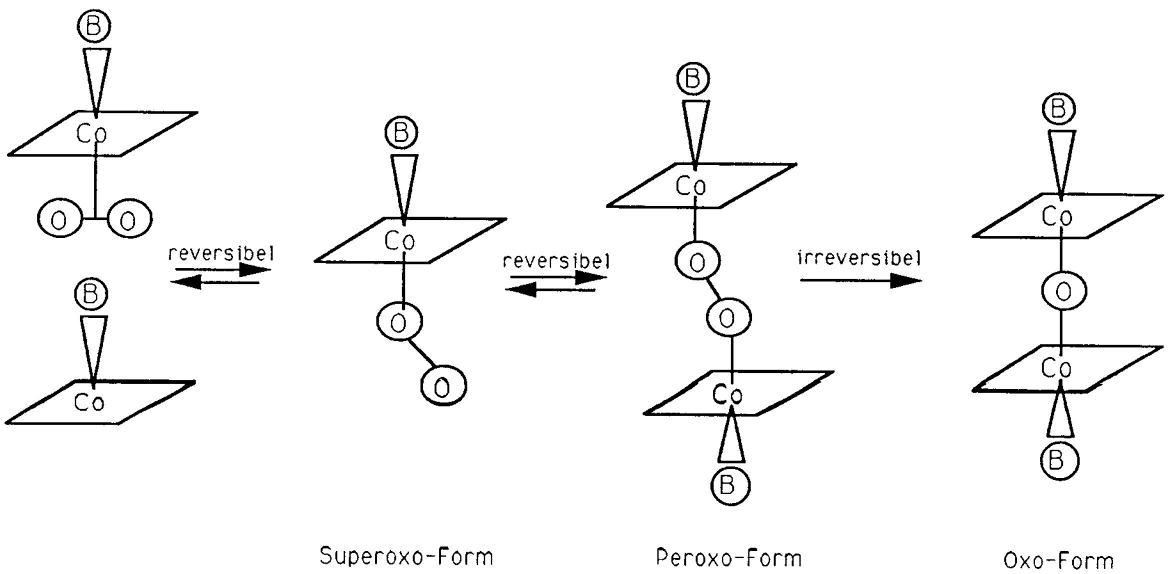
reversibel
reversibel
irreversibel
Superoxo-Form
Peroxo-Form
Oxo-Form

Abb. 3.

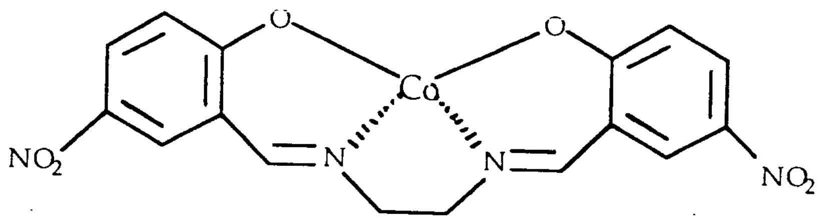

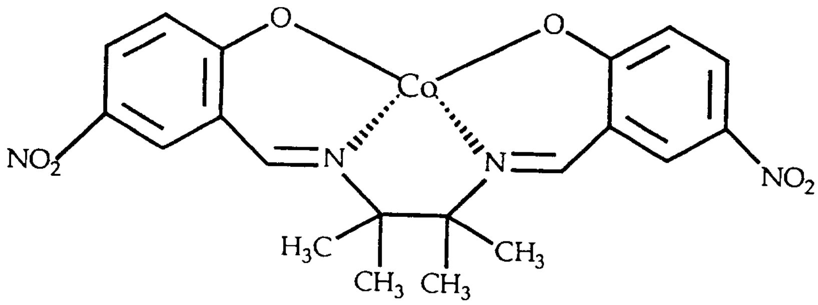

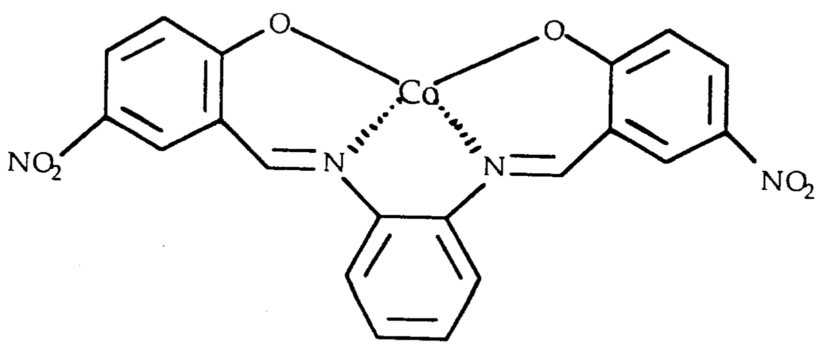

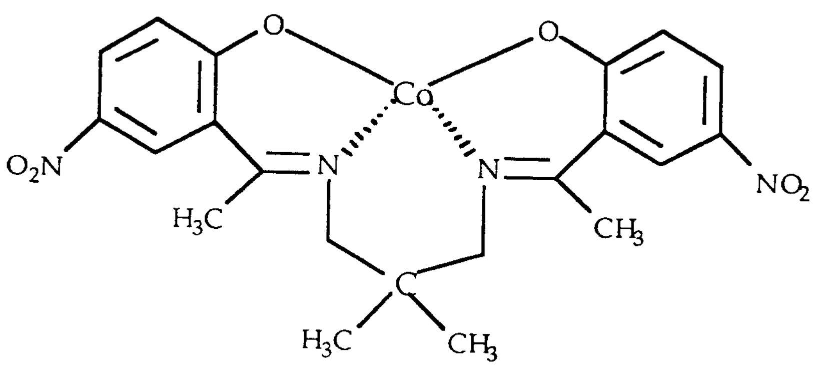

Abb. 4.

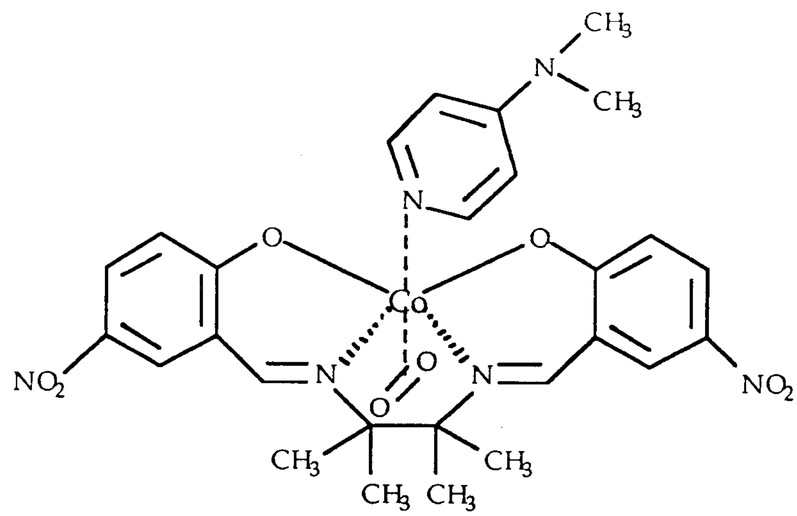

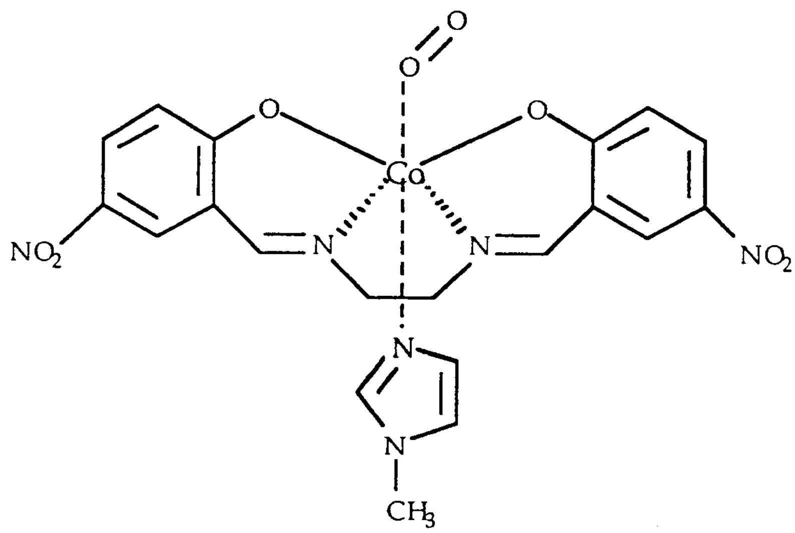

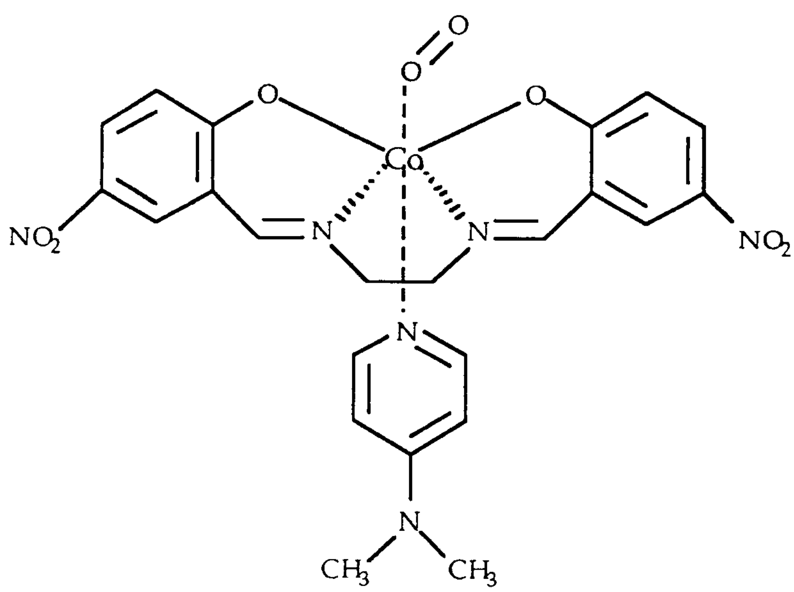

Abb.5.

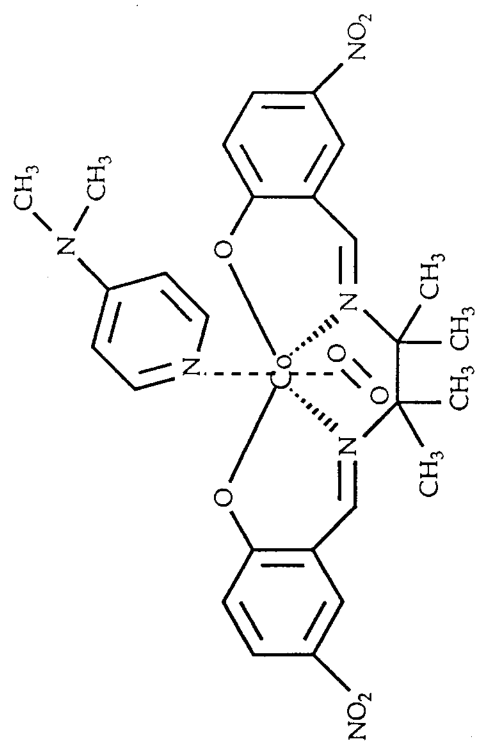

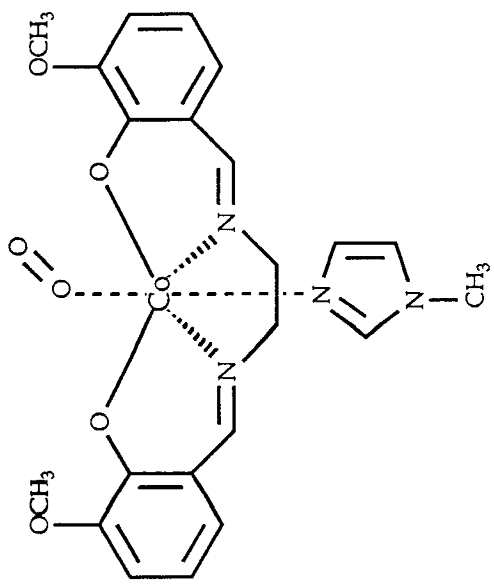

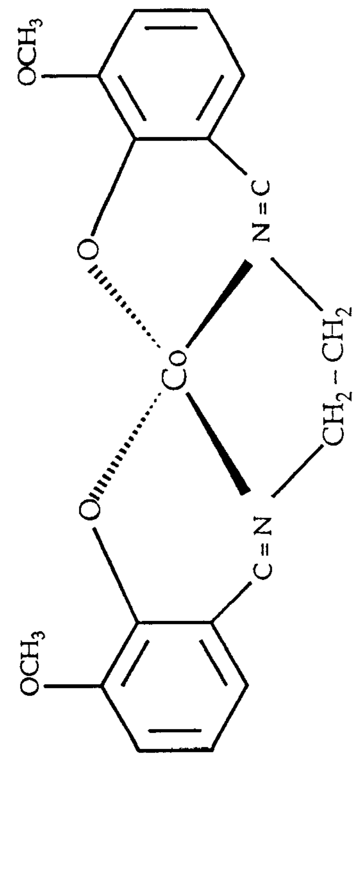

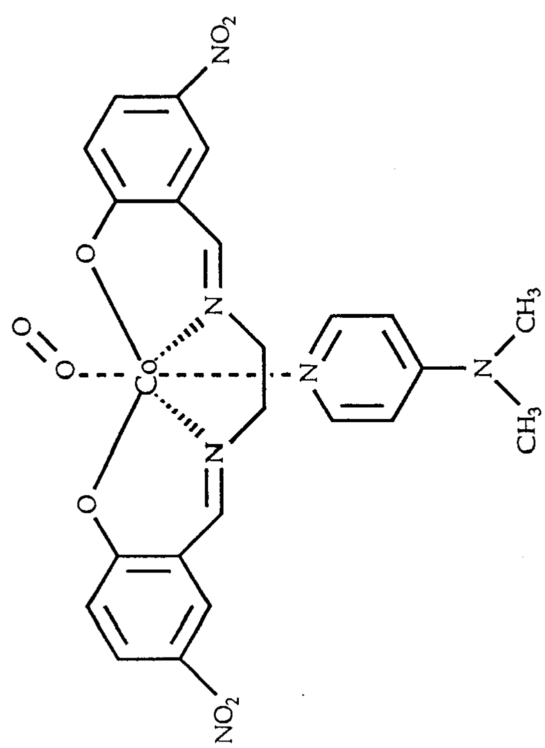

Abb.6.